# EUROPEAN PATENT APPLICATION

(11) **EP 1 577 289 A1**
(43) Date of publication of application: **21.09.2005**
(21) Application number: 04006461.0
(22) Date of filing: 18.03.2004
(51) Int. Cl.: C07C 233/00, C07C 237/00

(54) **Non-glycosylated/-glycosidic/-peptidic small molecule selectin inhibitors for the treament of inflammatory disorders**

(71) Applicant: Revotar Biopharmaceuticals AG, 16761 Henningsdorf (DE)
(72) Inventor: Kranich, Remo, Dr., 13503 Berlin (DE); Aydt, Ewald Mirko, Dr., 10247 Berlin (DE)
(74) Representative: Huhn, Michael

(57) **Abstract**

The compounds having the general structure of formula (I) 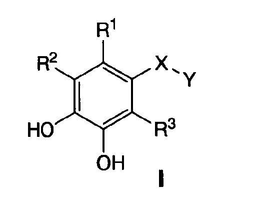 wherein the symbols, indices and substituents have the following meaning
if R²=OH and R³=H then R¹=H, CN, NO₂, CF₃, F, Cl, Br, I, CH₃ or
if R³=OH and R²=H then R¹=H, CN, NO₂, CF₃, F, Cl, Br, I, CH₃, Et, n-Pr, i-Pr, n-Bu, i-Bu, t-Bu, phenyl, thienyl, furyl, thiazolyl or
if R³=OH and R¹=H then R²=H, CN, NO₂, CF₃, F, Cl, Br, I, CH₃, Et, n-Pr, i-Pr,
n-Bu, t-Bu, phenyl, thienyl, furyl, thiazolyl
then X is e.g. 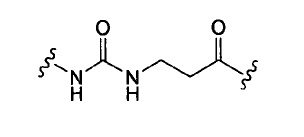 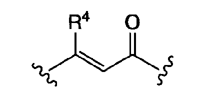 with R⁴ being H, CH₃, CH₂CH₃
or 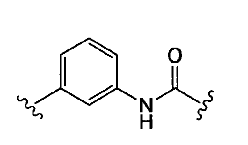 or 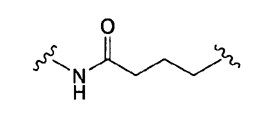 and Y being 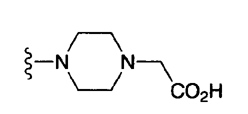 or 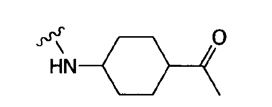 and the pharmaceutically acceptable salts, esters or amides and prodrugs of the above identified compounds of formula (I). The compounds are applied to modulate the in-vitro and in-vivo binding processes mediated by E-, P- or L-selectin binding.

## Description

Compounds and methods are provided, which modulate the in-vitro and in-vivo binding processes mediated by E-selectin, P-selectin or L-selectin binding. More specifically, selectin inhibitors and their use are described, wherein the selectin inhibitors that inhibit the selectin-mediated function comprise polyhydroxy phenyl subunits.

When a tissue has been invaded by a microorganism/is infected or has been damaged, the inflammatory process directs leukocytes and other immune system components to the site of infection or injury. Within this process, leukocytes (white blood cells) play a major role in the engulfment or digestion of microorganisms. Thus, the recruitment of leukocytes to infected or damaged tissue is critical for mounting an effective immune defense. Generally, white blood cells are found circulating through the bloodstream. To migrate from the blood stream into the affected tissue, the white blood cells must be able to recognize the invaded or damaged tissue and be able to bind to the wall of the capillary (endothelium) near the affected tissue and diffuse through the capillary into the affected tissue. Therefore, leukocytes have to roll and adhere to the endothelial cell wall. This cell adhesion event is one of the most important aspects of the inflammatory response. The first steps of this cell adhesion are mediated by members of the selectin family. The selectin family of vascular adhesion molecules is comprised of three structurally related calcium-dependent carbohydrate binding cell surface proteins, P-, L- and E-selectin. E-selectin is expressed only on inflamed endothelium, P-selectin is expressed on inflamed endothelium as well as on platelets, and has much structural similarity to E-selectin. L-selectin is expressed on leukocytes and also has much structure similarity to P- and E-selectins. The selectins are transmembrane proteins and are composed of an amino terminal lectin domain, an epidermal growth factor (EGF)-like domain, a variable number of complement receptor-related repeats, a hydrophobic domain spanning region and a cytoplasmic domain. The binding interactions which lead to the adhesion of the leukocytes appear to be mediated by contact of the lectin domain of the selectins and various carbohydrate ligands on the surface of the leukocytes. All three selectins can bind to the carbohydrate sialyl Lewis^{x}, a glycosyl moiety present on the surface of most/certain leukocytes. In case of P- and L-selectin, a distinct protein liband has been described [R.P. McEver; R.D. Cummings; *J.Clin.Invest.;* 1997; 100; 485-492], the so-called PSGL-1 (P-selectin glycoprotein ligand-1), which contributes to a high affinity selectin binding by its sLe^{x} moiety as well as by parts of its peptide components, sulphated tyrosine [R.P. McEver; *Ernst Schering Res. Found. Workshop;* 2004; 44; 137-147]. PSGL-1 is one of the most important selectin ligands binding with highest affinity to P-selectin, but it also binds to E-and L-selectin. It is expressed as homodimeric sialomucin on leukocytes and platelets where it is upregulated during inflammation.

In contrast to their low basal expression, E- and P-selectin expression is also upregulated during inflammation, leading to a substantial recruitment of leukocytes into the inflamed tissue. Although selectin-mediated cell adhesion is required for fighting infection, there are situations in which such cell adhesion is undesirable or excessive, resulting in tissue damage instead of repair. In the case of many acute as well as chronic inflammatory disorders (e.g., asthma, chronic obstructive pulmonary disease (COPD), psoriasis, etc.) an association between infiltration of activated leukocytes into the tissue simultaneously with a marked elevation of tissue expression of corresponding adhesion molecules, particularly E- and P-selectin, has been demonstrated. Such abnormal white blood cell infiltration, caused by extensive selectin expression may also play a role in transplant and graft rejection. Also the process of blood clotting is further promoted by leukocyte-leukocyte and leukocyte-platelet binding, which occurs because leukocytes possess as well L-selectin as its corresponding ligand PSGL-1 and can thus interact with themselves. via PSGL-1, and they can also bind to platelets which carry P-selectin.

D. Bock et al., New Drugs, p. 28 to 30 (28.04.2003) describe the role of selectin antagonists, especially bimosiamose, as inhibitor of selectins being an inflammation target:

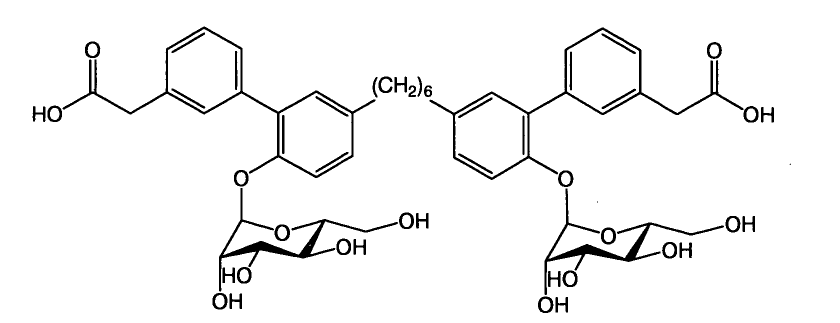

As additional targets MIF (Macrophage Migration Inhibitory Factor) and JAM-1 (Junctional Adhesion Molecule 1) are mentioned.

EP 0 758 243 B1 describes Mannopyranosyloxy-phenyl-benzoic acid or similar acids as components in a medicine for treating or preventing diseases, characterized by the binding of E-, P- and/or L-selectin to a sialyl-Lewis x (sLe^{x}) or sialyl-Lewis a (sLe^{a}) presented on a cell surface through the inhibition of such binding. Besides inflammatory diseases, other diseases like septic shock, psoriasis, rheumatoid arthritis, reperfusion injury and cancer are mentioned.

From EP 0 840 606 B1 compounds like bimosiamose are known, e.g., 1,6-Bis-[3-(carboxymethylphenyl)-4-(2-α-D-mannopyranosyloxy)phenyl]hexan and derivatives thereof including heptan, butan and pentan derivatives. Again, their use for inhibiting the selectin binding to sLe^{x} or sLe^{a} are mentioned.

A different method for inhibiting the binding between a first cell having a selectin and a second cell having a ligand for selectin in vitro is described in EP 0 902 681 B1 in permitting a lipid composition to interact with the first cell. A proportion of the lipids has an attached saccharide like fucooligosaccharides or lactose, a different proportion has, e.g., a second attached acidic monosaccharide, further proportions are crosslinked and a proportion without attached saccharides have an acidic group being negatively charged at neutral pH. These lipid compositions are used in local alterations in the adherence of leukocytes or cancer cells to vascular endothelium, platelets or lymphatic tissue.

EP 1 288 222 A1 describes compounds with affinity to P-selectin being peptides with a specific amino acid sequence comprising, e.g., valine, tryptophan, aspartic acid, and D- or L-glutamic acid. Medicaments comprising these peptides are said as inhibiting leukocyte binding to platelets and/or endothelial cells. Similar peptides are known from WO-A 03/020753.

The disalicylates and disalicylate-based C-glycosides of WO-A 99/29706 act as selectin-ligand structural mimetics in medicaments. They may lack the sialic acid and/or fucose of the natural selectin ligand being sLe^{x}. A typical compound is

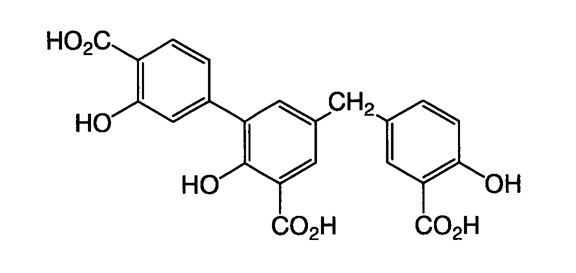

The 5-membered heterocycles of WO-A 00/33836 exhibit inhibitory activity against the selectins; typical structures are 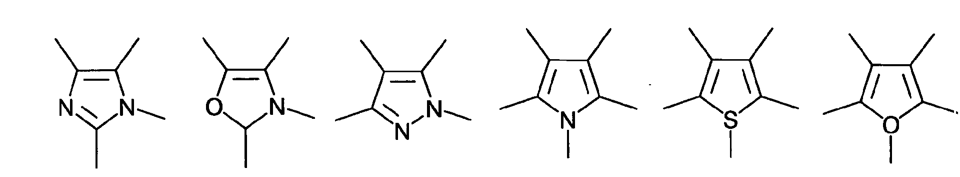 with substituents like a moiety containing a terminal carboxylic acid group as a non-N substituent, a hydrophobic moiety such as a functionalized alkyl chain as a non-N substituent and a functionalized aryl group which also may be an N-substituent.

WO-A 01/89531 provides methods for identifying agents which interact with (activate or inhibit) the crystal and three-dimensional structures of a) the lectin and EGF-like (LE) domains of P-selectin, of b) P-selectin LE and E-selectin LE each complexed with sLe^{x}, and c) P-selectin LE complexed with a functional PSGL-1 peptide modified by both tyrosine sulfation and sLe^{x}.

The compounds for modulating in vitro and in vivo processes mediated by selectin binding according to WO-A 03/097658 are benzyl amino sulfonic acids linked to carbohydrate or glycomimetic. A typical compound would be 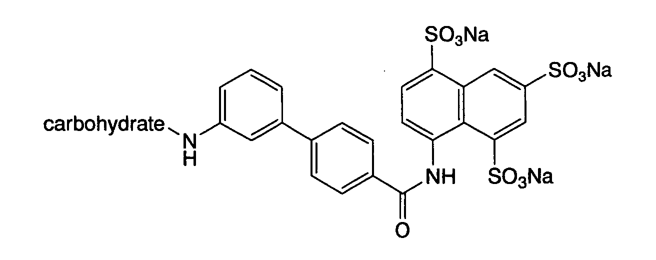 C.C.M.Appeldoom et al. in JPC Papers in Press, published January 13, 2003, with the title "Optimization of a P-selectin binding peptide" report on peptides containing a Glu-Trp-Val-Asp-Val consensus motif to which gallic acid or 1,3,5-benzenetricarboxylic acid at the N-terminus have been added as substituents. C-terminus modifications had little to no influence on the affinity of the core peptide. The two typical substituents are 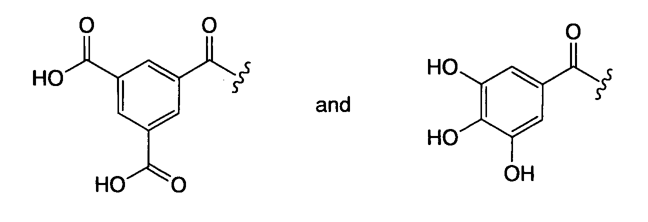 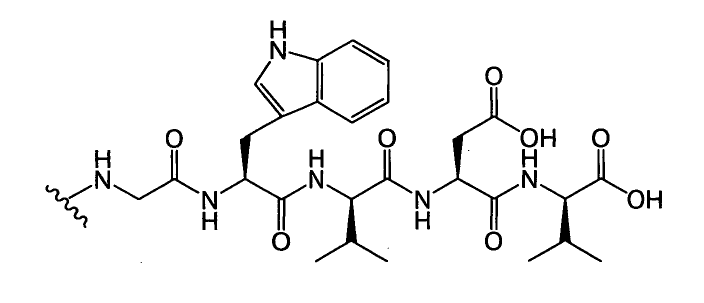 is a typical peptide rest.

In such a very complex pharmaceutical field, there is a strong medical need in the art for indentifying further inhibitors of selectin-mediated function, e.g. of selectin-dependent cell adhesion, and for the development of methods employing such compounds to inhibit conditions associated with excessive selectin activity. Most of the available pharmaceutical therapies e.g. of psoriasis or asthma, which are available on the market comprise mostly corticosteroids or NSAIDs (non steroidal anti-inflammatory drugs) which have several serous drawbacks/side effects, and target different steps of the inflammatory cascade.

Object of the invention is to provide new compounds, especially non-glycosylated/glycosidic relatively small molecules, which are able to inhibit selectin and which have less negative side effects during their in-vitro or in-vivo application. Furthermore, the said molecules should show a good bioavailability when applied as an active ingredient in a medicine.

The present invention provides compounds having the general structure of formula (I). 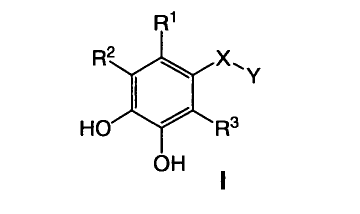 wherein the symbols, indices and substituents have the following meaning
if R²=OH and R³=H then R¹=H, CN, NO₂, CF₃, F, Cl, Br, I, CH₃ or
if R³=OH and R²=H then R¹=H, CN, NO₂, CF₃, F, Cl, Br, I, CH₃, Et, n-Pr, i-Pr, n-Bu, i-Bu, t-Bu, phenyl, thienyl, furyl, thiazolyl or
if R³=OH and R¹=H then R²=H, CN, NO₂, CF₃, F, Cl, Br, I, CH₃, Et, n-Pr, i-Pr, n-Bu, t-Bu, phenyl, thienyl, furyl, thiazolyl
-X- =
(a) 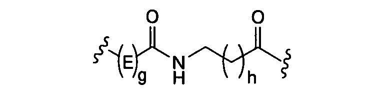 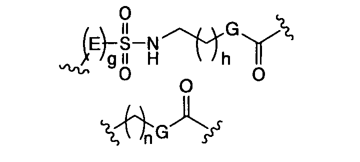 with -E- being -NH-, -(CH₂-)ₖNH-; -G- being -(NH-)ₘ and g = 0,1; h = 1, 2, 3; k= 1, 2, 3; m = 0,1; n = an integer from 1 to 8
(b) 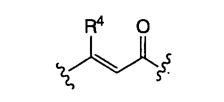 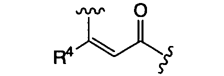 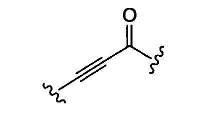 with R⁴ being H, CH₃, CH₂CH₃
(c) 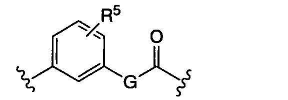 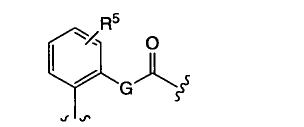 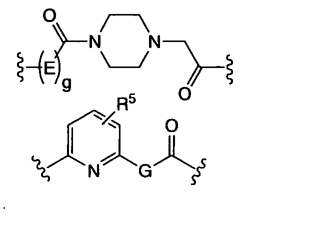 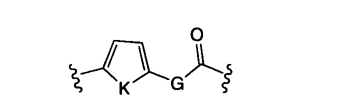 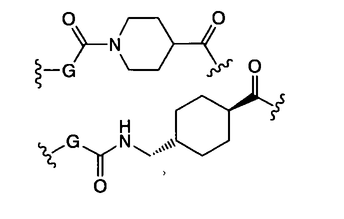 with R⁵ being H, NO₂, CF₃, F, Cl, Br, I, CN, CH₃ and -K- being -S- or -O-
(d) 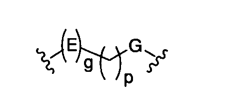 with p being an integer from 2 to 8
(e) 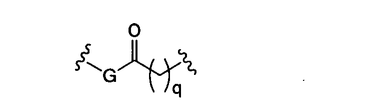 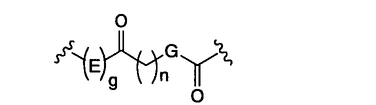 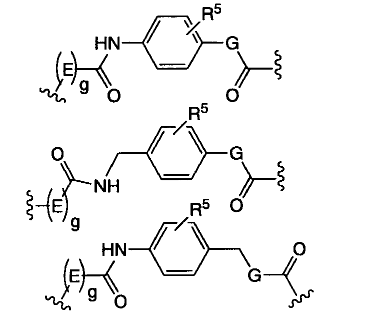 with q being an integer from 1 to 9 and r being an integer from 1 to 3
(f) 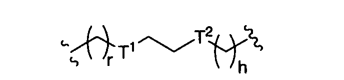 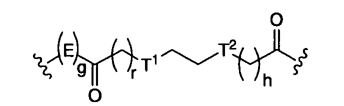 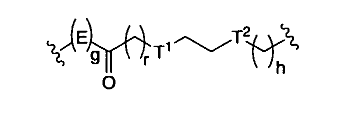 with -T¹-, -T²-being -E-, -K- or -(N-alkyl)--Y = 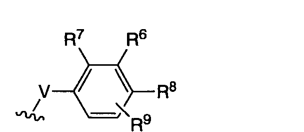 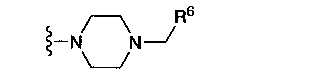 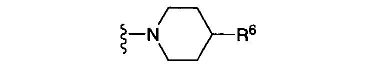 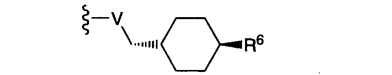 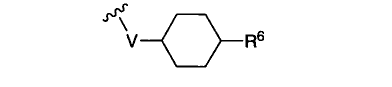 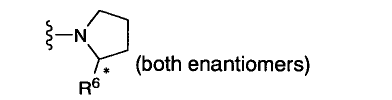 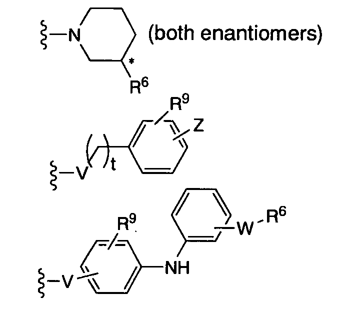 with -V- being -(NH-)ₛ- with s being 0 or 1,

R⁶ being CO₂H, CO₂Alkyl, CO₂Aryl, CO₂NH₂, CO₂Aralkyl, SO₃H, SO₂NH₂, PO(OH)₂, 1-H-tetrazolyl-, CHO, COCH₃, CH₂OH, NH₂, NHAlkyl, N(Alkyl)Alkyl', OCH₃, CH₂OCH₃, SH
R⁷ independently from R⁶ being H, CH₃, CH₂CH₃, CF₃, F, Cl, Br, I, CN, NO₂ and
R⁸ independently from R⁶ and R⁷ being H, CH₃, CH₂CH₃, CF₃, F, Cl, Br, I, CN, NO₂, R⁶
R⁹ being H, NO₂, CF₃, F, Cl. Br, I, CN, CH₃, OCH₃, SH
t being 0,1,2
and -W- = -(CH₂-)ᵥ, *cis*-CH=CH- or *trans-*CH=CH-, and v beine 0,1,2 -Z =

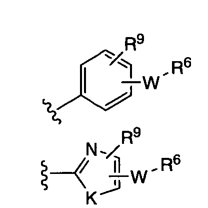

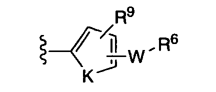

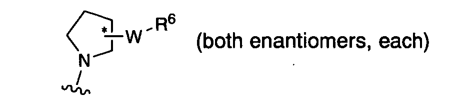

-W-R⁶

Preferred compounds are those of formulas (A) and (B) 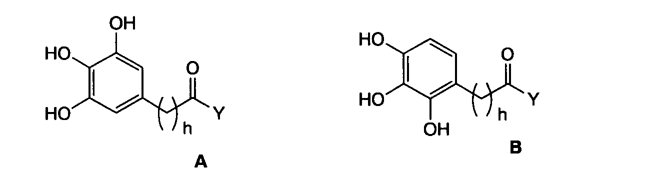 wherein -Y is (Q=CH, N) 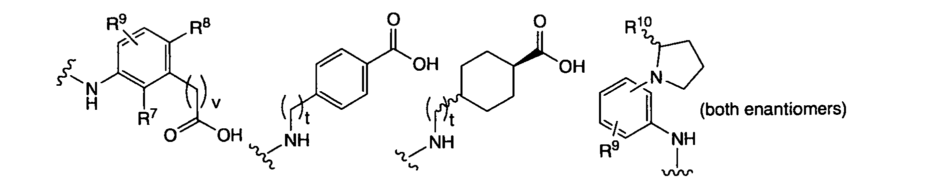 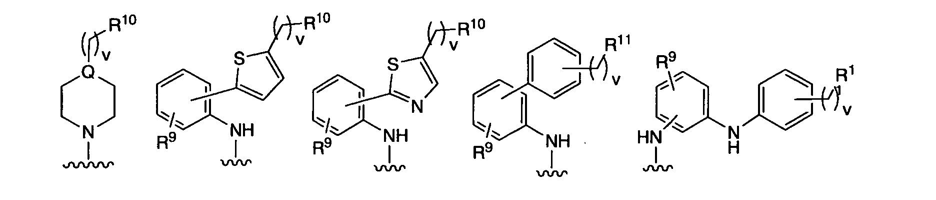 with R¹⁰ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH,
R¹¹ being CO₂H, CO₂alkyl, CO₂aryl, CO₂NH₂, CO₂aralkyl, SO₃H, SO₂NH₂, PO(OH)₂, 1-H-tetrazolyl, CHO, COCH₃, OH, NH₂, NHalkyl, N(alkyl)alkyl', OCH₃, SH

Further particularly preferred compounds include the following formulas (C) and (D) 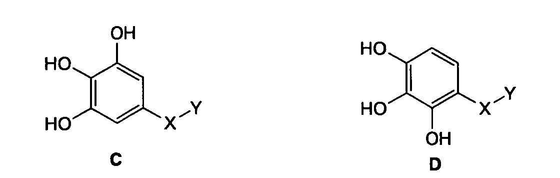 wherein Y is like relating to formulas (A) and (B) and X is:

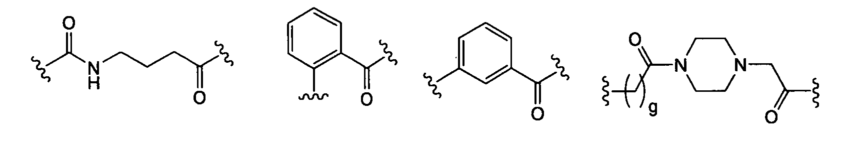

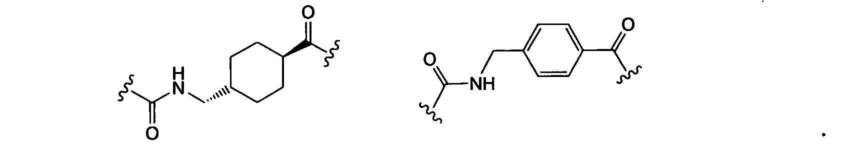

Also part of the invention are the pharmaceutically acceptable salts, esters or amides and prodrugs of the above-identified compounds of formula (I).
The present invention also provides pharmaceutical compositions comprising at least one compound of the formula (I) and a pharmaceutically acceptable carrier which is useful in a medicine. These compounds present the ability of inhibiting cell adhesion and inhibit selectin-[as well as PSGL-1-mediated] binding. The compounds have the ability to inhibit the interaction of selectins with sLe^{x} / sLe^{a} and also the interaction between selectins and tyrosinesulfate residues.
In a preferred variant the invention provides pharmaceutical compositions comprising at least one compound of formula (A) or formula (B) or formula (C) or formula (D) and a pharmaceutically acceptable carrier. The present invention further provides a method of inhibiting the binding of P-selectin, L-selectin or E-selectin to sLe^{x} or sLe^{a} and tyrosinesulfate residues comprising the step of administering to a patient an effective amount of at least one compound having the structure of formula (I) to inhibit the binding of P-, E- or L-selectin to sLe^{x} or sLe^{a} and tyrosinesulfate.

It has been found that compounds having the formula (I) shown above act to inhibit E, P, or L-selectin binding.
As used herein the term "alkyl" shall mean a monovalent straight chain or branched chain group of 1 or 2 or 3 or 4 or 5 or 6 or 7 or 8 or 9 or 10 or 11 or 12 carbon atoms including, but not limited to, methyl, ethyl, n-propyl, isopropyl, n-butyl, sec-butyl, tert-butyl and the like.
The term "aryl" shall mean carbocyclic and heterocyclic aromatic groups including, but not limited to, phenyl, 1-naphthyl, 2-naphthyl, fluorenyl, (1,2)-dihydronaphthyl, indenyl, indanyl, thienyl, benzothienyl, thienopyridyl and the like.
The term "aralkyl" (also called arylalkyl) shall mean an aryl group appended to an alkyl group including, but not limited to, benzyl, 1-naphthylmethyl, 2-naphthylmethyl, fluorobenzyl, chlorobenzyl, bromobenzyl, iodobenzyl, alkoxybenzyl (wherein "alkoxy" means methoxy, ethoxy, isopropoxy, n-butoxy, sec-butoxy, tert-butoxy an the like), hydroxybenzyl, aminobenzyl, nitrobenzyl, guanidinobenzyl, fluorenylmethyl, phenylmethyl(benzyl), 1-phenylethyl, 2-phenylethyl, 1-naphthylethyl and the like.

The term "pharmaceutically acceptable salts, esters, amides and prodrugs" as used herein refers to those carboxylate salts, amino acid addition salts, esters, amides and prodrugs of the compounds of the present invention which are, within the scope of sound medical judgement, suitable for use in contact with tissues of patients without undue toxicity, irritation, allergic response and the like, commensurate with a reasonable benefit/risk ratio, and effective for their intended use, as well as the zwitterionic forms, where possible, of the compounds of the present invention. The term "salts" refers to the relatively non-toxic, inorganic and organic acid addition salts of the compounds of the present invention. These salts can be prepared *in situ* during the final isolation and purification of the compounds or by separately reacting the purified compounds in its free form with a suitable inorganic or organic acid or base and isolating the salt thus formed. Representative salts of the compounds of the present invention include the hydrobromide, hydrochloride, sulfate, bisulfate, nitrate, acetate, oxalate, valerate, palmitate, stearate, laurate, borate, benzoate, lactate, phosphate, tosylate, citrate, maleate, fumarate, succinate, tartrate, naphthylate, mesylate, glucoheptonate, lactiobionate, laurylsulphonate salts and the like. These may include cations based on the alkali and alkalineearth metals, such as sodium, lithium, potassium, calcium, magnesium and the like, as well as non-toxic ammonium, quaternary ammonium and amine cations including, but not limited to, ammonium, tetramethylammonium, tetraethylammonium, methylamine, dimethylamine, trimethylamine, triethylamine, ethylamine, and the like.
Examples of the pharmaceutically acceptable, non-toxic esters of the compounds of this invention include C₁, C₂, C₃, C₄, C₅ and C₆ alkyl esters wherein the alkyl group is a straight or branched chain. Acceptable esters also include C₅, C₆ and C₇ cycloalkyl esters as well arylalkyl esters such as, but not limited to benzyl. C₁, C₂, C₃, C₄, C₅ and C₆ alkyl ester are preferred. Esters of the compounds of the present invention may be prepared according to conventional methods.

Examples of pharmaceutically acceptable, non-toxic amides of compounds of this invention include amides derived from ammonia, primary C₁, C₂, C₃, C₄, C₅ and C₆ alkyl amines and secondary C₁, C₂, C₃, C₄, C₅ and C₆ dialkyl amines wherein the alkyl groups are straight or branched chains. In the case of secondary amines the amine may also be in the form of a 5 or 6 membered heterocycle containing one nitrogen atom. Amides derived from ammonia, C₁, C₂ and C₃ alkyl primary amides and C₁ to C₂ dialkyl secondary amides are preferred. Amides of the compounds of the present invention may be prepared according to conventional methods.

The term "prodrug" refers to one or more compounds that are rapidly transformed *in vitro* and from a non-active to active state *in vivo* to yield to the parent compound of the above formula (I), for example by hydrolysis in blood or *in vivo* metabolism.
The present invention also provides for pharmaceutically active compositions that contain the compounds of the present invention. It is also contemplated that pharmaceutically active compositions may contain a compound of the present invention or other compounds that inhibit or compete with E-selectin or P-selectin or L-selectin binding.
Pharmaceutically active compositions of the present invention comprise a physiological carrier and a compound of formula (I). The pharmaceutical compositions of the present invention may include one or more of the compounds having the above structure (I) formulated together with one or more non-toxic, physiologically acceptable carriers, adjuvants or vehicles, which are collectively referred to herein as carriers, for parenteral injection, for oral administration in solid or liquid form, for rectal or topical administration and the like.
The compositions can be administered to humans and animals either orally, rectally, parenterally (intravenously, intramuscularly, or subcutaneously), intracisternally, intravaginally, interperitoneally, locally (powders, ointments or drops), or as a buccal or by inhalation (nebulized, or as nasal sprays).
Compositions suitable for parenteral injection may comprise physiologically acceptable sterile aqueous or nonaqueous solutions, dispersion, suspensions or emulsions and sterile powders for reconstitution into sterile injectable solution or dispersion. Examples of suitable aqueous and nonaqueous carriers, diluents, solvents or vehicles include water, ethanol, polyol, (propylene glycol, polyethylene glycol, glycerol and the like), suitable mixtures thereof, vegetable oils (such as olive or canola oil) and injectable organic esters such as ethyl oleate. Proper fluidity can be maintained, for examples, by the use of a coating such as lecithin, by the maintenance of the required particle size in the case of dispersions and by the use of surfactants.
These compositions may also contain adjuvants such as preserving, wetting, emulsifying, and dispersing agents. Prevention of the actions of microorganisms can be ensured by various antibacterial and antifungal agents, for examples, parabens, chlorobutanol, phenol, sorbic acid, and the like. It may also be desirable to include isotonic agents, for examples sugars, sodium chloride and the like. Prolonged absorption of the injectable pharmaceutical form can be brought about by the use of agents delaying absorption, for examples aluminium monostearate and gelatin.
If desired, and for more effective distribution, the compounds can be incorporated into slow or timed release or targeted delivery systems such as polymer matrices, liposomes, and microspheres. They may be sterilized, for example, by filtration through a bacteria-retaining filter, or by incorporating sterilizing agents in the form of sterile water, or some other sterile injectable medium immediately before use.
Solid dosage forms for oral administration include capsules, tablets, pills, powders and granules. In such solid dosage forms, the active compound or a prodrug ester is admixed with at least one inert customary excipient (or carrier) such as sodium citrate or dicalcium phosphate or (i) fillers or extenders, as for example, starches, lactose, sucrose, glucose, mannitol and silicic acid, (ii) binders, as for example, carboxymethylcellulose, alginates, gelatine, polyvinylpyrrolidone, sucrose and acacia, (iii) humectants, as for example, glycerol, (div disintegrating agents, as for example, agar-agar, calcium carbonate, potato or tapioca starch, aliginic acid, certain complex silicates and sodium carbonate, (v) solution retarders, as for examples, paraffin, (vi) absorption accelerators, as for example, quaternary ammonium compounds, (vii) wetting agents, as for examples, cetyl alcohol and glycerol monostearate, (viii) adsorbents, as for example, kaolin and bentonite, and (ix) lubricants, as for example, talc, calcium stearate, magnesium stearate, solid polyethylene glycols, sodium lauryl sulfate and mixtures thereof. In the case of capsules, tablets, and pills, the dosage forms may also comprise buffering agents.
Solid compositions of a similar type may also be employed as fillers in soft and hard-filled gelatine capsules using excipients as lactose or milk sugars as well as high molecular polyethylene glycols and the like.
Solid dosage forms such as tablets, dragées, capsules, pills and granules can be prepared with coatings and shells, such as enteric coatings and others well known in the art. They may contain opacifying agents, and can also be of such compositions that they release the active compound or compounds in a certain part of the intestinal tract in a delayed manner. Examples of embedding compositions that can be used are polymeric substances and waxes.
The active compounds can also be in microencapsulated form, if appropriate, with one or more of the above-mentioned excipients
Liquid dosage forms for oral administration include pharmaceutically acceptable emulsions, solutions, suspensions, syrups and elixirs. In addition to the active compounds, the liquid dosage forms may contain inert diluents commonly used in the art such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, cannola oil, caster oil and sesame seed oil, glycerol, tetrahydrofurfuryl alcohol, polyethylene glycols and fatty acid esters of sorbitan or mixtures of these substances, and the like.
Besides such inert diluents, the compositions can also include adjuvants, such as wetting agents, emulsifying and suspending agents, sweeting, flavouring and perfuming agents.
Suspensions, in addition to the active compounds, may contain suspending agents, for example, ethoxylated isostearyl alcohols, polyoxyethylene sorbitol and sorbitan esters, microcrystalline cellulose, aluminium metahydroxide, bentonite, agar-agar, tragacanth or mixtures of these substances and the like.
Compositions for rectal administrations are preferably suppositories, which can be prepared by mixing the compounds of the present invention with suitable nonirritating excipients or carriers such as cacao butter, polyethylene glycol or a suppository wax, which are solid at ordinary temperatures but liquid at body temperature and therefore melt in the rectal or vaginal cavity and release the active component.
Dosage forms for topical administration of a compound of this invention include ointments, powder, sprays and inhalants.
The active component is admixed under sterile conditions with a physiologically acceptable carrier and any needed preservatives, buffers or propellants as may be required. Ophthalmic formulations, eye ointments, suspensions, powder and solutions are also contemplated as being within the scope of this invention.
The compounds of the present invention can also be administrated in the form of liposomes. As is known in the art, liposomes are generally derived from phospholipids or other lipid substances. Liposomes are formed by mono or multilamellar hydrated liquid crystals that are dispersed in an aqueous medium. Any non-toxic, physiologically acceptable metabolized lipid capable of forming liposomes can be used. The present compositions in liposome form can contain, in addition to the selectin binding inhibitors of the present invention, stabilizers, preservatives, excipients and the like. The preferred lipids are the phospholipids and the phosphatidyl cholines (lecithins), both natural and synthetic. Methods to form liposomes are well known in the art.
Actual dosage levels of active ingredient in the composition of the present invention may be varied so as to obtain an amount of active ingredient that is effective to obtain the desired therapeutic response for a particular composition and method of administration. The selected dosage level, therefore, depends on the desired therapeutic effect, on the route of administration, on the desired duration of treatment and other factors.
The total daily dosage of the compounds on this invention administered to a host in single or divided doses may be in the range up to 50 mg per kilogram of body weight. Dosage unit compositions may contain such submultiples thereof as may be used to make up the daily dosage. It will be understood, however, that the specific dose level for any particular patient, whether human or other animal, will depend upon a variety of factors including the body weight, general health, sex diet, time and route of administration, rates of absorption and excretion, combination with other drugs and the severity of the particular disease being treated.
In particular, the compounds of the present invention may be used to treat a variety of diseases relating to inflammation and cell-cell recognition and adhesion. For example, the compounds of the present invention may be administrated to a patient to treat COPD, acute respiratory distress syndrome (ARDS), Crohn's disease, septic shock, chronic inflammatory diseases such as psoriasis, atopic dermatitis, and rheumatoid arthritis, and reperfusion tissue injury that occurs following heart attacks, strokes, atherosclerosis, and organ transplants, traumatic shock, multi-organ failure, autoimmune diseases like multiple sclerosis, asthma and inflammatory bowel disease. In each case, an effective amount of the compounds of the present invention is administered either alone or as part of a pharmaceutically active composition to a patient in need of such treatment. It is also recognized that a combination of the compounds may be administered to a patient in need of such administration. The compounds of the present invention may also be administered to treat other diseases that are associated with cell-cell adhesion. As the present compounds inhibit the binding of E-selectin or P-selectin or L-selectin, any disease that is related to this interaction may potentially be treated by the inhibition of this binding interaction.
In addition to being found on some white blood cells, sLe^{a} is found on various cancer cells, including lung and colon cancer cells. It has been suggested that cell adhesion involving sLe^{a} may be involved in the metastasis of certain cancers and inhibitors of sLe^{a} binding might be useful in treatment of some forms of cancer.

Many of the compounds of the present invention may be synthesized according to the following general synthetic schemes. 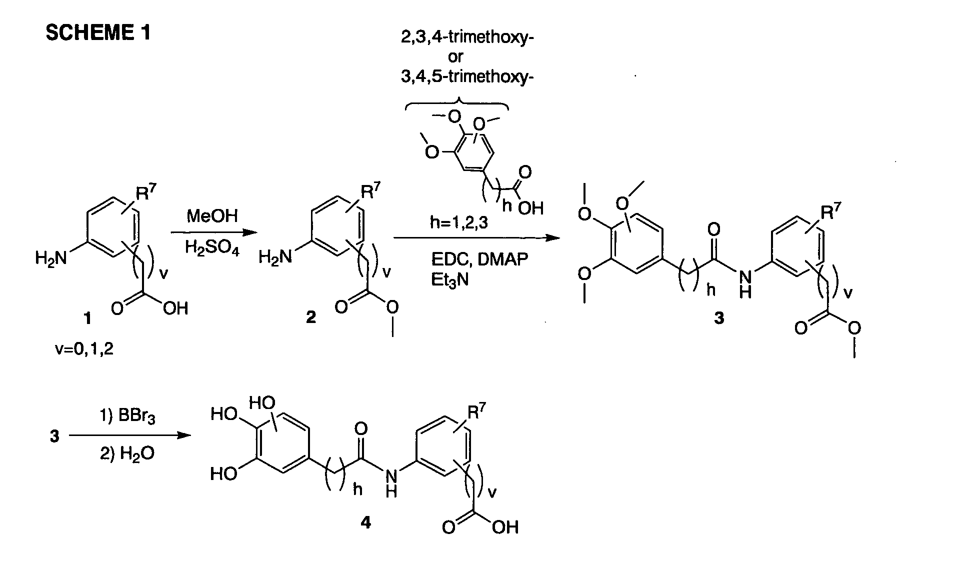 In SCHEME 1 an amino acid of type (1) is reacted to the corresponding methyl ester (2) under heating with acidic methanol. Ester (2) is reacted with a trimethoxy-phenyl-alkylic acid under state-of-the-art conditions (i.e. N'-(3-dimethylaminopropyl)-N-ethyl carbodiimide (EDC), triethylamine and 4-dimethylaminopyridine (DMAP) in a chlorinated solvent) to the amide (3). Alternatively diisopropyl carbodiimide (DIC) and hydroxybenzotriazole (HOBt) may be used for this reaction step. Amide (3) is converted to acid (4) by treating it with an excess of boron tribromide at -78°C up to rt in a halogenated solvent followed by aqueous workup. However, in some cases it may be necessary to hydrolize the ester (3) with i.e. aqueous lithium hydroxide in acetonitrile before treating it with boron tribromide. The synthesis sequence shown in SCHEME 1 leading to compounds like (4) is not only reduced to the Y-H building blocks like (1) but may be generally applied to all other Y-H type building blocks leading to compounds of type (A) and (B) as shown in the paragraph before. 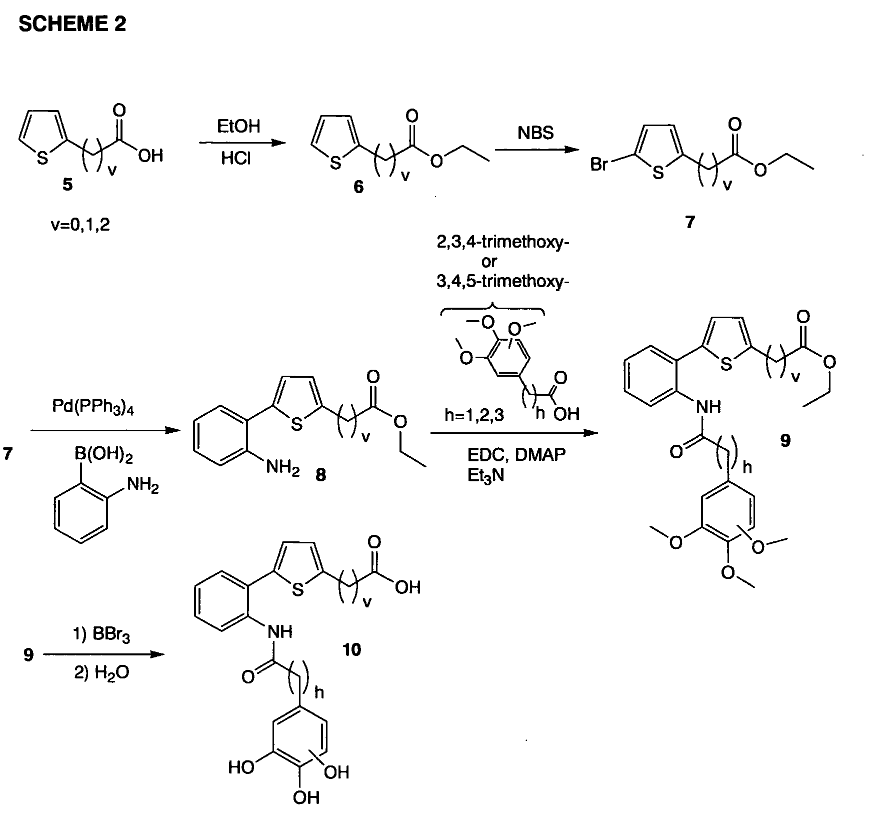 In SCHEME 2 a carboxy substituted thiophene like (5) is reacted to the corresponding ethyl ester (6) under heating in acidic ethanol. Ester (6) is brominated with N-bromosuccinimide in anhydrous chloroform and glacial acetic acid to give (7) which is further reacted with 2-Amino-benzeneboronic acid under a state-of-the-art Suzuki transformation (i.e. Tetrakis(triphenylphosphine)-palladium, aqueous sodium carbonate, ethanol, toluene) to the biaryl (8). Biaryl (8) is reacted with a trimethoxy-phenyl-alkylic acid, EDC, triethylamine and DMAP in a chlorinated solvent to the amide (9). Alternatively DIC and HOBt may be used for this reaction step. Amide (9) is converted to acid (10) by treating it with an excess of boron tribromide at -78°C up to rt in a halogenated solvent followed by aqueous workup. However, in some cases it may be necessary to hydrolize the ester (9) with i.e. aqueous lithium hydroxide in acetonitrile before treating it with boron tribromide. 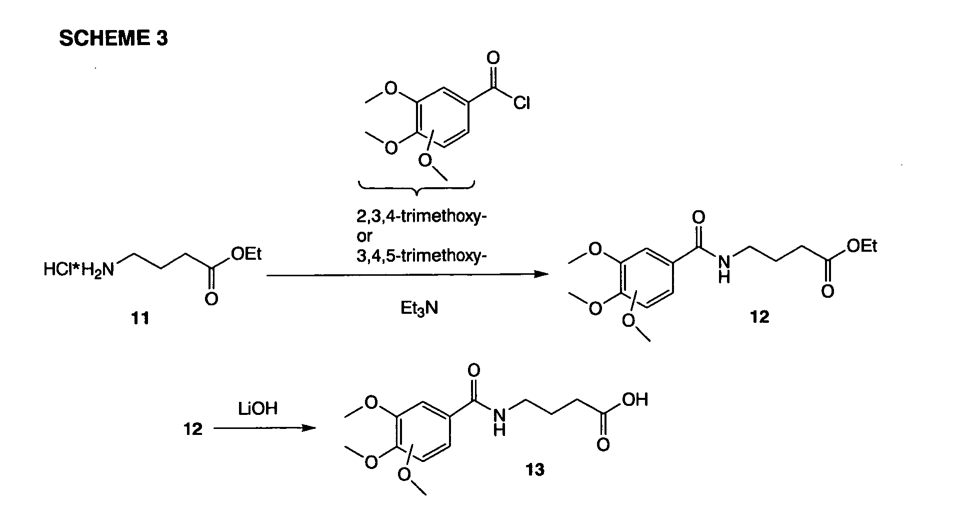 In SCHEME 3 4-Amino-butyric acid hydrochloride (11) is reacted at rt with an trimethoxybenzoic acid under basic conditions (triethylamine in anhydrous dichloromethane) to the amide (12) which is further hydrolized with aqueous lithium hydroxide in acetonitrile to give a building block like (13). 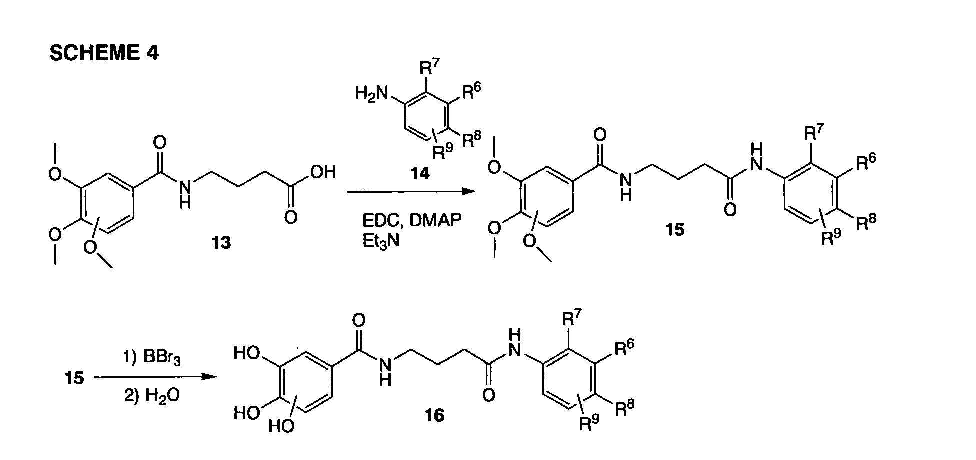 In SCHEME 4 an acid like (13) is reacted with an aniline of general type (14) under state-of-the-art conditions (i.e. EDC, triethylamine and DMAP in a chlorinated solvent) to form the amide (15). Alternatively DIC and HOBt may be used for this reaction step. Amide (15) is converted to acid (16) by treating it with an excess of boron tribromide at -78°C up to rt in a halogenated solvent followed by aqueous workup.
In case that R⁶ and/or R⁸ contain carboxylic acid functionalities, those are protected as their corresponding methyl or ethyl esters before and hydrolized afterwards to release the carboxylic acid functionalities. The ester hydrolysis was done whether with LiOH in MeCN or under treatment with BBr₃ in followed by addition of water.
The synthesis sequence shown in SCHEME 4 leading to compounds like (16) is not only reduced to X-Y-H and Y-H building blocks like (14) but may be generally applied to all other X-Y-H and Y-H type building blocks leading to compounds of type (C) and (D) as shown in the paragraphs before. 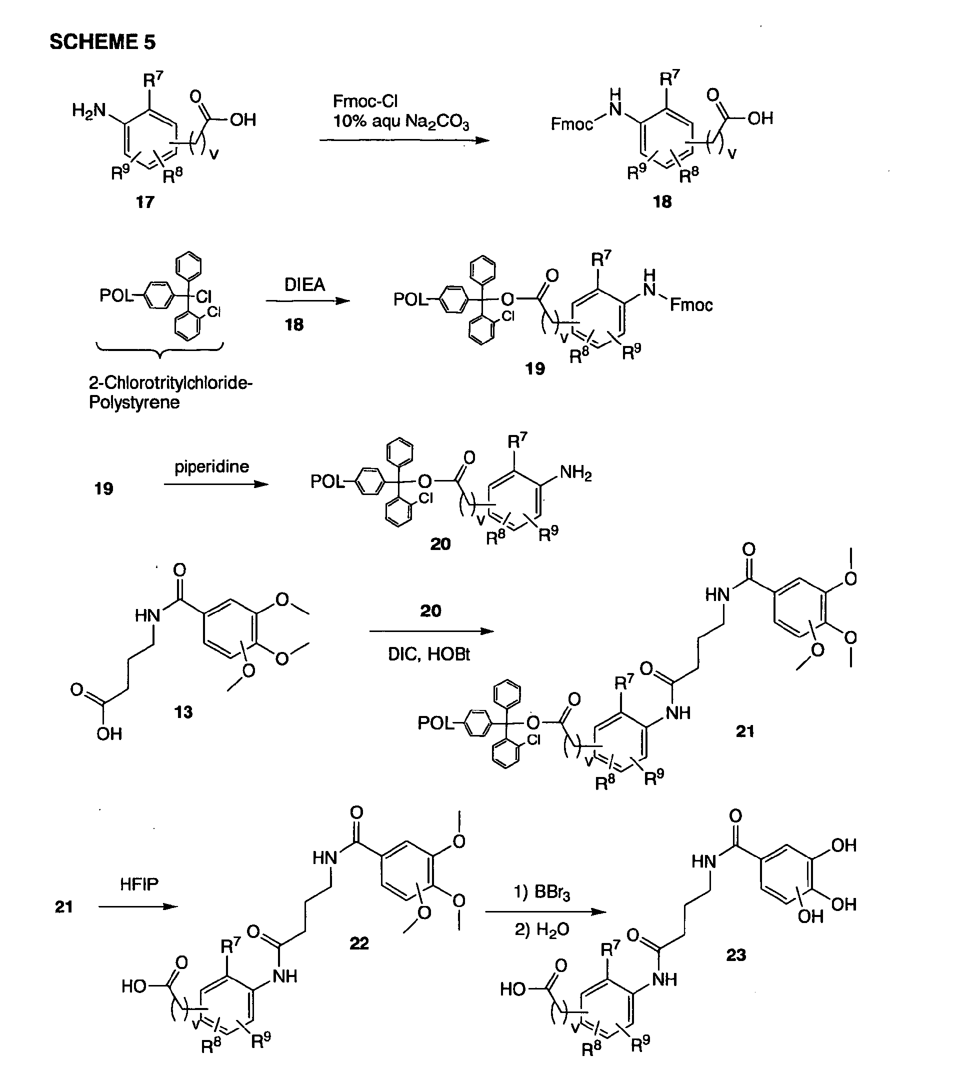 For compounds of type (23) containing a carboxylic acid functionality a synthesis sequence according to SCHEME 5 may be applied. An aniline of type (17) is reacted at rt with 9-fluorenylmethoxycarbonyl chloride (Fmoc-Cl) in 1,4-dioxane and aqueous sodium carbonate to the corresponding Fmoc-protected compound (18). Acid (18) is immobilized on solid support by esterification with 2-Chlorotritylchloride-polystyrene and Huenig's base in anhydrous dichloromethane and N,N-dimethylformamide at rt to give (19) which is further treated with piperidine in N,N-dimethylformamide. The resulting compound (20) is reacted then with acid (13), DIC and HOBt in N,N-dimethylformamide at rt. The resulting amide (21) is treated with 1,1,1,3,3,3-Hexafluoro-2-propanol in dichloromethane at rt to cleave (22) from solid support. Following treatment of (22) with an excess of boron tribromide in dichloromethane at -78°C up to rt and aqueous workup provides the desired compound (23).
The present invention is furthermore illustrated by the following representative examples.

### EXAMPLE 1

### {3-[3-(2,3,4-Trihydroxy-phenyl)-propionylamino]-phenyl}-acetic acid (27)

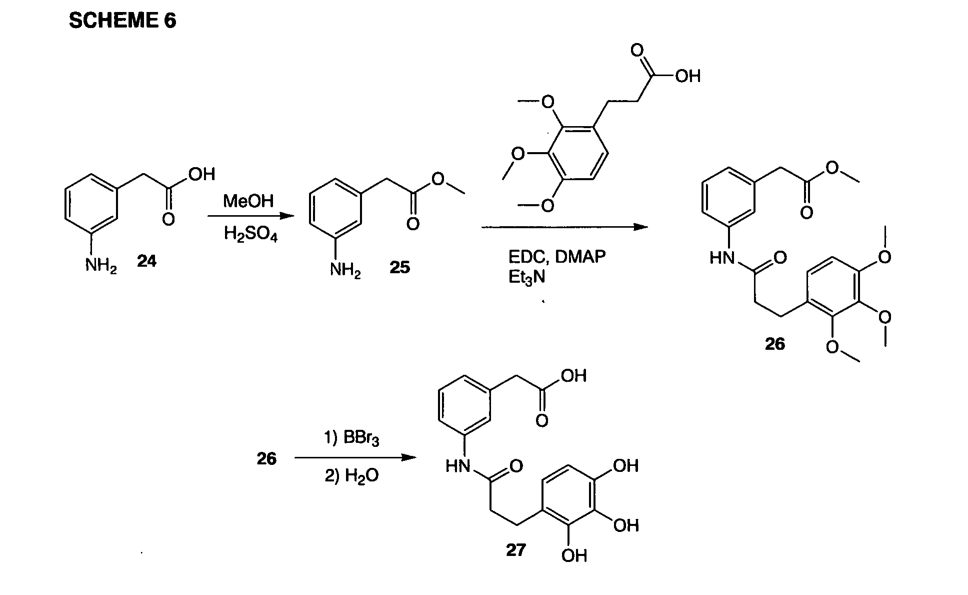

**Step 1**: Dissolve (3-Amino-phenyl)-acetic acid ((24),700mg, 4.63mmol) in MeOH (21mL) and add conc. sulfuric acid (0.27mL, 5.09mmol). Stir the reaction mixture for 2d under reflux. Cooled mixture to room temperature (rt), remove solvent under reduced pressure and prepurify the residue by flushing it over a short pad of silica gel using EtOAc. Remove solvent again and partitione the residue between EtOAc and saturated aqu. NaHCO₃ (1+1). Extracte the aqueous layer 3 times with EtOAc, washe the combined organic layers with brine and dried with Na₂SO₄. Remove solvent under reduced pressure and dry the residue without further purification in oil pump vacuum to obtain product (25) as a light yellow oil (708mg, 92%). ¹H NMR (400MHz, CDCl₃): 3.51 (s, 2 H); 3.67 (s, 3 H); 6.57 (dd, 1 H, *J*₁ = 7.8Hz, *J*_{*2*} = 1.BHz); 6.60 (br.Ψt, 1 H, *J* = 1.8Hz); 6.65 (br.d, 1 H, *J* ≈ 7.8Hz); 7.08 (Ψt, 1 H, *J* = 7.8Hz).

**Step 2:** (The following reaction is done in an anhydrous N₂ atmosphere.) Dissolve EDC hydrochloride (187mg, 0.98mmol) and triethylamine (0.14mL, 1.00mmol) in anhydrous dichloromethane (3.5mL) and stir for 5min at rt. Added 3-(2,3,4-Trimethoxy-phenyl)-propionic acid (234mg, 0.97mmol) and DMAP (12mg, 0.10mmol) and stir for 10min. Added ester (25) (107mg, 0.65mmol) and stir the reaction solution overnight at rt.
Hydrolize the reaction solution with saturated aqu. NH₄Cl followed by water, separate layers, extracte aqu. layer with dichloromethane (3 times) and washe the combined organic layers with water and brine and dry with Na₂SO₄. Remove solvent under reduced pressure.
Purify crude product by preparative radial chromatography (silica gel 60PF, EtOAc/CyH 1+1) to obtain product (26) as a white solid (209mg, 83%). [K. C. Nicolaou; P. S. Baran; Y.-L. Zhong; K. Sugita; *J. Am. Chem. Soc.;* **2002;** *124;* 10; 2212-2220]. ¹H NMR (400MHz, CDCl₃): 2.62 (t, 2 H, *J* = 7.5Hz); 2.95 (t, 2 H, *J* = 7.5Hz); 3.58 (s, 2 H); 3.67 (s, 3 H); 3.82 (s, 3 H); 3.84 (s, 3 H); 3.91 (s, 3 H); 6.59 (d, 1 H, *J* = 8.6Hz); 6.86 (d, 1 H, *J* = 8.6Hz); 6.98 (br.d, 1 H, *J* = 7.8Hz); 7.32 (Ψt, 1 H, *J* ₌ 7.8Hz); 7.38 (br.d, 1 H, *J* = 7.8Hz); 7.41 (br.s, 1 H).

**Step 3:** (The following reaction is done in an anhydrous N₂ atmosphere.) Dissolve (26) (139mg, 0.36) in anhydrous dichloromethane (1.8mL), cooled to -78°C (acetone/ dry ice bath) and add slowly BBr₃ (0.51mL, 5.39mmol). Stir the reaction mixture for additional 30min at -78°C. Remove cooling bath and stir the reaction mixture for 3h at rt.
Cool reaction mixture to 0°C, add slowly water (0.50mL) under vigorous stiring followed by dichloromethane (1.0mL) and methanol (2.0mL). Extract the mixture with EtOAc (3 times) and dry the combined organic layers with Na₂SO₄. Remove solvent under reduced pressure and purify crude product by preparative RP HPLC (gradient, water/MeCN 95:5) to obtain {3-[3-(2,3,4-Trihydroxy-phenyl)-propionylamino]-phenyl}-acetic acid (27) as a white solid (69mg, 57%). [T. P. Kogan; B. Dupré; H. Bui; K. L. McAbee; J. M. Kassir; I. L. Scott; X. Hu; P. Vanderslice; P. J. Beck; R. A. F. DixonJ. *Med. Chem.;* **1998;** 41; 1099-1111]. ¹H NMR (400MHz, CD₃OD): 2.67 (t, 2 H, *J* = 7.6Hz); 2.92 (t, 2 H, *J* = 7.6Hz); 3.61 (s, 2 H); 6.31 (d, 1 H, *J* = 8.3Hz); 6.50 (d, 1 H, *J* = 8.3Hz); 7.05 (br.d, 1 H, *J* = 7.8Hz); 7.28 (Ψt, 1 H, *J* = 7.8Hz); 7.48 (br.d, 1 H, *J* = 7.8Hz); 7.49 (br.s, 1 H).

### EXAMPLE 2

### (5-{2-[2-(2,3,4-Trihydroxyphenyl)-acetylamino]-phenyl}-thiophen-2-yl]acetic acid (34)

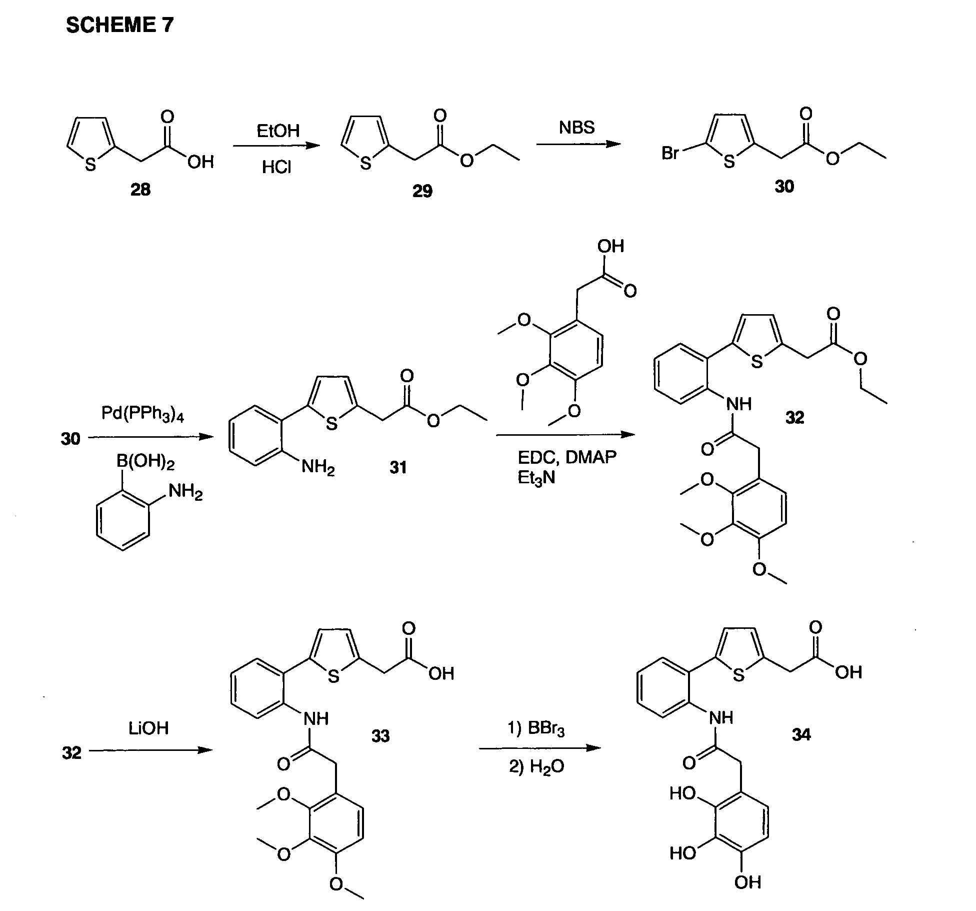

**Step 1:** Dissolve Thiophene-2-yl-acetic acid (28) (2.44g, 17.1mmol) in ethanol (35mL) and add fuming aqu. hydrochloric acid (few drops). Stir the reaction mixture for 19h at 70 °C. Cool mixture to rt, remove solvent under reduced pressure and resolve the residue in EtOAc. Wash this organic layer 3 times with 5% aqu. Na₂CO₃ and extract the combined aqueous layer 3 times with EtOAc. Wash the combined organic layers with brine and dry with Na₂SO₄. Remove solvent under reduced pressure and dry the residue without further purification in oil pump vacuum to obtain product (29) as a light brown oil (2.78g, 95%). [J. Kunes; V. Balsanek; M. Pour; V. Buchta; *Collect. Czech. Chem. Commun.,* **2001,** *66;* 12; 1809-1830]. ¹H NMR (400MHz, CDCl₃): 1.26 (t, 3 H, *J* = 7.1 Hz); 3.81 (s, 2 H); 4.17 (q, 2 H, *J* = 7.1 Hz); 6.91-6.96 (m, 2 H); 7.20 (d, 1H, *J* = 4.8 Hz).

**Step 2:** (The following reaction is done in an anhydrous N₂ atmosphere.) Dissolve ester (29) (1.30g, 7.64mmol) in anhydrous chloroform (6.0mL) and glacial acetic acid (6.0mL), add N-Bromosuccinimide (1.39g, 7.79mmol) in portions and stir the mixture for 23h at rt. The mixture is diluted with an equal volume of water, the organic layer separated and washe with a 1M aqu. NaOH, water, again with 1M aqu. NaOH and water (2 times). Finally wash the organic layer with brine and dry with Na₂SO₄. Remove solvent under reduced pressure. Purify crude product by preparative radial chromatography (silica gel 60PF, CyH/EtOAc 5+1] to obtain product (30) as an impured (according to NMR: 20% sideproduct) orange liquid (1.61g, 85%) which is used without any further purification. [P. M. Jackson; C.J. Moody; P. Sha; J. *Chem. Soc. Perkin Trans. 1;* **1990;** 2909-2918]. ¹H NMR (400MHz, CDCl₃): 1.26 (t, 3 H, *J* = 7.1 Hz), 3.73 (s, 2 H); 4.17 (q, 2 H, *J* = 7.1 Hz); 6.67 (d, 1 H, *J* = 3.5 Hz); 6.88 (d, 1 H, *J* = 3.5 Hz).

**Step3:** (The following reaction is done in an oxygenfree N₂ atmosphere.) ethanol (1.47mL), Tetrakis-(triphenylphosphine)-palladium(0) (59.0mg, 2.5mol%) and aqu. Na₂CO₃ (1.60g, 5.60mmol; presolved in 2.0mL H₂O) aree subsequently added to dissolved 2-Aminobenzeneboronic acid (341mg, 2.20 mmol) in toluene (16mL). The reaction mixture is degassed 5 times and flooded with N₂ again. Add bromide (30) (498mg, 2.00mmol) and rinse with toluene (4.5 mL), degas again (5 times) and stir the reaction solution 21h at 100°C. Partition the reaction solution between EtOAc and brine (1+1) and extract the separated aqueous layer 3 times with EtOAc. Wash combined organic layer with brine and dry with Na₂SO₄. Remove solvent under reduced pressure and purify the crude product by preparative radial chromatography (silica gel 60PF, CyH/EtOAc 6+1, later 3+1] to obtain product (31) as a light yellow solid (300mg, 57%). [N. Miyaura; A. Suzuki; *Chem. Rev.;* **1995**; 95; 2457]. ¹H NMR (400MHz, CDCl₃): 1.28 (t, 3 H, J = 7.1 Hz); 3.82 (s, 2 H); 4.19 (q, 2 H, J = 7.1 Hz); 6.77-6.84 (m, 2 H); 6.91 (d, 1 H, J = 3.5 Hz); 7.04 (d, 1 H, J = 3.5 Hz); 7.13 (td, 1 H, J = 7.8 Hz, 1.3 Hz); 7.25 (d, 1 H, J = 7.8 Hz).

**Step4:** (The following reaction is done in an anhydrous N₂ atmosphere.) Suspend EDC hydrochloride (86.3mg, 0.45mmol) in anhydrous dichloromethane (1.4mL), add triethylamine (0.063mL, 0.45mmol) and stir for 10min at rt. Add 2-(2,3,4-Trimethoxy-phenyl)-acetic acid (74.7mg, 0.33mmol) and DMAP (3.7mg, 0.03mmol) and stir for 15min. Added ester (31) (64,9mg, 0.30mmol) and stir the reaction solution 22h at rt. Partition the reaction solution between dichloromethane and water (1+1), separate layers and extract aqu. layer with dichloromethane (3 times). Wash the combined organic layer with brine and dry with Na₂SO₄. Purify crude product by preparative radial chromatography (silica gel 60PF, CyH/EtOAc 3+2) to obtain product (32) as yellow oil (118mg, 84%). ¹H NMR (400MHz, CDCl₃): 1.29 (t, 3 H, J = 7.1 Hz); 3.58 (s, 2 H); 3.74 (s, 3 H); 3.78 (s, 3 H); 3.79-3.80 (m, 2 H); 3.86 (s, 2 H); 4.20 (q, 2 H, J = 7.1 Hz); 6.58 (d, 1 H, J = 8.6 Hz); 6.59 (d, 1 H, J = 3.5 Hz); 6.75 (d, 1 H, J = 3.5 Hz); 6.85 (d, 1 H, J = 8.6 Hz); 7.05 (t, 1 H, J =7.8 Hz); 7.26 (dd, 1 H, J = 7.8 Hz, 1.3 Hz); 7.30 (td, 1 H, J = 7.8 Hz, 1.3 Hz); 7.90 (br.s; 1 H), 8.38 (d, 1 H, J = 8.3 Hz).

**Step 5:** Dissolve ester (32) (118mg, 0.25mmol) in methanol (8.0mL), add a 1M aqu. LiOH solution (1.76mL, 1.76mmol) and stir 20h at rt. Remove solvent under reduced pressure und partition residue between CHCl₃ and 0.5M HCl (1+1). Separate the aqueous layer and extract 3 times with CHCl₃. Wash the combined organic layer with brine and dry with Na₂SO₄. Remove solvent under reduced pressure and dry the residue without further purification in oil pump vacuum to obtain crude product (33) as light brown foam (120mg, quant.). ¹H NMR (400MHz, CDCl₃): 3.58 (s, 2 H); 3.73 (s, 3 H); 3.78 (s, 3 H); 3.85 (s, 2 H); 3.86 (s, 3 H); 6.58-6.61 (m, 1 H); 6.59 (d, 1 H, J = 8.3 Hz); 6.77 (d, 1 H, J = 3.5 Hz); 6.86 (d, 1 H, *J* = 8.3 Hz); 7.06 (t, 1 H, *J* = 7.8 Hz); 7.22-7.27 (m, 1 H); 7.31 (td, 1 H, *J* = 7.8 Hz, 1.3 Hz); 7.86 (br.s, 1 H); 8.37 (d, 1 H, *J* = 8.3 Hz).

**Step 6**: (The following reaction is done in an anhydrous N₂ atmosphere.) Dissolve (33) (118mg, 0.27mmol) in anhydrous dichloromethane (2.5mL), cool to -78°C (acetone/ dry ice) and add slowly a 1M BBr₃ solution in dichloromethane (1.61mL, 1.61mmol). Stir the reaction mixture for additional 30min at -78°C. Remove cooling bath and stir the reaction mixture for 4h at rt. Cool reaction mixture to 0°C, add slowly water (1.00mL) under vigorous stirring. Partition the reaction mixture between EtOAc and water (1+1). Extract the separated aqueous layer with EtOAc (2 times) and wash the combined organic layer with brine and dry with Na₂SO₄. Remove solvent under reduced pressure and purify crude product by preparative RP HPLC (gradient, water/CH₃CN 95:5) to obtain (5-{2-[2-(2,3,4-Trihydroxyphenyl)-acetylamino]-phenyl}-thiophen-2-yl] acetic acid (34) as a light brown foam (53mg, 50%). ¹H NMR (400MHz, CDCl₃): 3.54 (s, 2 H); 3.82 (s, 2 H); 6.34 (d, 1 H, *J* = 8.3 Hz); 6.49 (d, 1 H, *J* = 8.3 Hz); 6.76 (d, 1 H, *J* = 3.3 HZ); 6.81 (d, 1 H, *J* = 3.3 Hz); 7.18 (t, 1 H, *J* = 7.6 Hz); 7.32 (t, 1 H, *J* = 7.8 Hz); 7.39 (d, 1 H, *J* = 7.3 Hz); 7.90 (d, 1 H, *J* = 8.1 Hz); 8.20 (br.s, 1 H).

### EXAMPLE 3

### 4-(3,4,5-Trihydroxy-benzoylamino)-butyric acid (36)

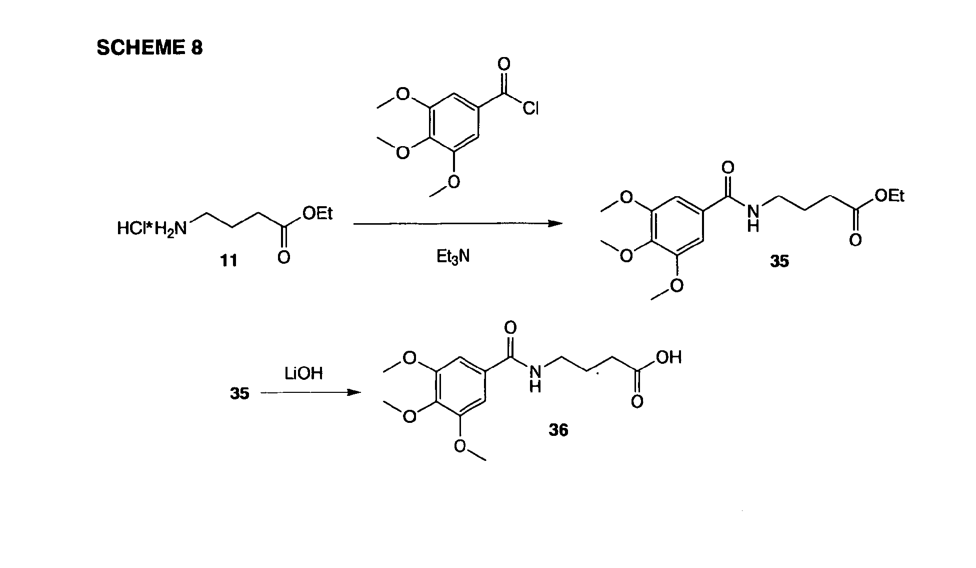

**Step 1:** (The following reaction is done in an anhydrous N₂ atmosphere.) Dissolve 4-amino-butyric acid ethyl ester hydrochloride (11) (3.98g, 23.74mmol) in anhydrous dichloromethane (120mL) at rt and add triethylamine (8.30mL, 59.55mmol). Stir the reaction solution for 15min at rt, add portionwise 3,4,5-trimethoxy-benzoyl chloride (6.58g, 28.53mmol) and stir for additional 24h at rt.
Hydrolize the reaction solution with methanol, filtrated the mixture through a short pad of silica gel with EtOAc/CyH (3+1). Purify crude product by flash chromatography (silica gel, EtOAc/CyH 2+1, later 3+1) to obtain (35) as a white solid (4.48g, 58%). ¹H NMR (400MHz, CDCl₃): 1.22 (t, 3 H, *J* = 7.1Hz); 1.95 (Ψquint, 2 H, *J* = 6.5Hz); 2.44 (t, 2 H, *J* = 6.5Hz); 3.48 (td, 2 H, *J*_{*1*} = 6.5Hz, *J*_{*2*} = 5.8Hz); 3.85 (s, 3 H); 3.89 (s, 6 H); 4.10 (q, 2 H, *J* = 7.1Hz); 6.72 (br.s, 1 H); 7.02 (s, 2 H).

**Step 2:** Dissolve (35) (4.48g, 13.79mmol) in acetonitile (100mL) at rt and add 1M aqu. LiOH (41.4mL, 41.4mmol). Stir reaction mixture for 18h at rt.
Hydrolize the reaction mixture (cooling bath) with 1M aqu. HCl (50mL). Extract the mixture with chloroform (3 times), wash the combined organic layers with brine and dry with Na₂SO₄ to obtain 4-(3,4,5-trihydroxy-benzoylamino)-butyric acid (36) as a white solid (4.07g, 99%). No further purification. ¹H NMR (400MHz, CDCl₃): 1.94 (Ψquint, 2 H, *J* = 6.7Hz); 2.46 (t, 2 H, *J* = 6.7Hz); 3.50 (br.td, 2 H); 3.85 (s, 3 H); 3.88 (s, 6 H); 6.61 (br.s, 1 H); 7.00 (s, 2 H).

### EXAMPLE 4

### 3-[4-(3,4,5-Trihydroxy-benzoylamino)-butyrylamino]-benzoic acid (38)

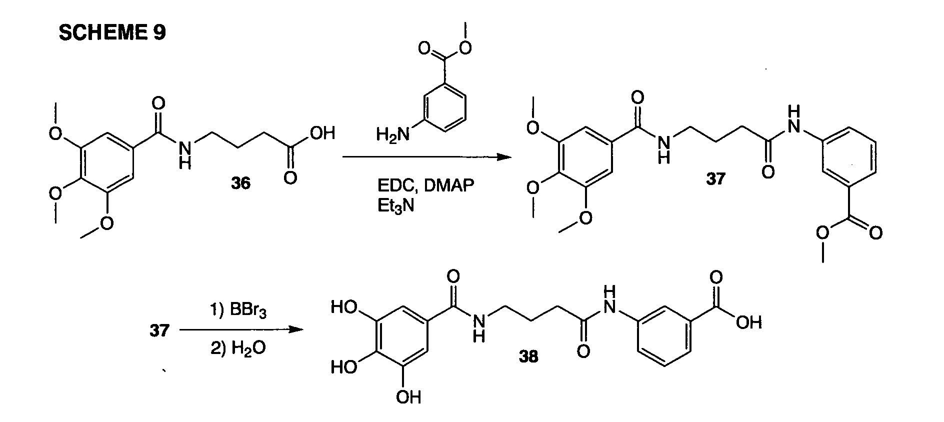

**Step 1:** (The following reaction is done in an anhydrous N₂ atmosphere.) Dissolve EDC x HCl (141mg, 0.74mmol) and Et₃N (0.105mL, 0.74mmol) in anhydrous DCM (4.0mL) and stir for 5min at rt. Added (36) (208mg, 0.70mmol) and DMAP (8.6mg, 0.07mmol) and stir for 5min. Add 3-Aminomethyl-benzoate (159mg, 1.05mmol) and stir the reaction solution for 21h at rt. Hydrolize the reaction solution with sat. aqu NH₄Cl and water, separate layers, extract aqu layer with EtOAc (3 times) and wash the combined organic layer with water and brine and dry with Na₂SO₄. Purified crude product by flash chromatography (silica gel 60, EtOAc/CyH 3+1, later EtOAc/MeOH 9+1) to obtain (37) as a white solid (219mg, 72%). ¹H NMR (400MHz, CDCl₃): 2.01-2.10 (br.m, 2 H); 2.52 (br.t, 2 H, *J* ca. 6Hz); 3.55-3.63 (br.m, 2 H); 3.85 (s, 6 H); 3.89 (s, 3 H); 7.02 (s, 2 H); 7.35 (dd, 1 H, *J*_{*1*} = 8.1Hz, *J*_{*2*} = 7.6Hz); 7.75 (d, 1 H, *J* = 7.6Hz); 7.80 (d, 1 H, *J* = 8.1Hz); 8.15 (s, 1 H); 8.55 (br.s, 1 H).

**Step 2:** (The following reaction is done in an anhydrous N₂ atmosphere.) Dissolve (37) (86mg, 0.2mmol) in anhydrous DCM (4.0mL) at -25°C and add dropwise BBr₃ (0.303mL, 3.20mmol). The reaction mixture is slowly warmed up to rt and stir for additional 6h at rt. Cool reaction mixture to 0°C, add dropwise water/THF (1+1, 1mL) followed by DCM (2mL). Stir overnight and add MeOH (2mL) to homogenize the mixture. Remove solvent under reduced pressure and purify crude product by preparative RP HPLC (gradient, water/MeCN 95:5) to obtain 3-[4-(3,4,5-Trihydroxy-benzoylamino)-butyrylamino]-benzoic acid (38) as a white solid (41mg, 55%). ¹H NMR (400MHz, CD₃OD): 2.02 (br.tt, 2 H*, J*_{*1*} *=* 7.1Hz, *J*₂ ca. 6.5Hz); 2.50 (t, 2 H, *J* = 7.1Hz); 3.46 (br.t, 2 H, *J* ca. 6.5Hz); 6.90 (s, 2 H); 7.43 (dd, 1 H, *J*_{*1*} = 8.1Hz, *J*_{*2*} = 7.6Hz); 7.78 (d, 1 H, *J* = 7.6Hz); 7.85 (d, 1 H, *J* = 8.1Hz); 8.26 (br.s, 1 H).

### EXAMPLE 5

### 4-{[4-(2,3,4-Trihydroxy-benzoylamino)-butyrylamino]-methyl}-cyclohexanecarboxylic acid (46)

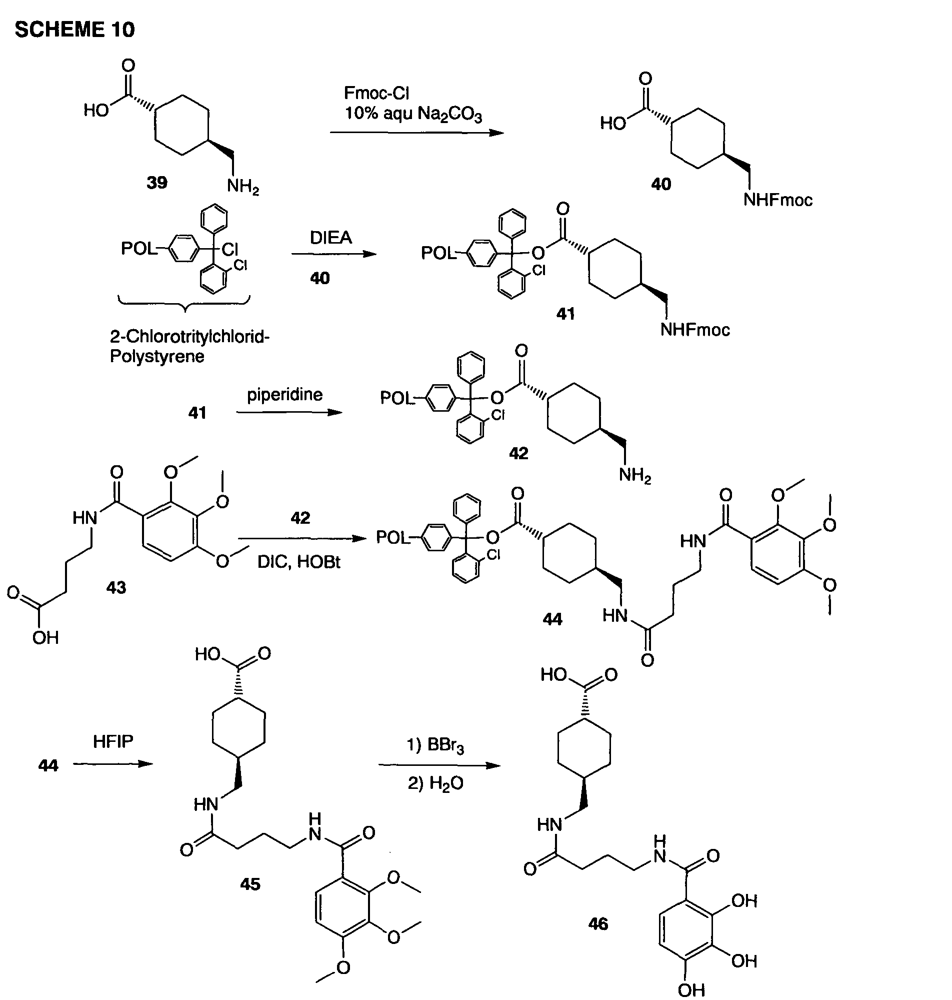

**Step 1**: Dissolve 4-Aminomethyl-cyclohexanecarboxylic acid ((39); 1.15g, 7.30mmol) in 1,4-dioxane (12.0mL) and 10% aqu. Na₂CO₃ (23.2mL) and add Fmoc-Cl (2.26g, 8.76mmol). Stir the reaction mixture for 2.5h at rt. Add 1M aqu HCl (42.0mL) to the mixture and extract with EtOAc (3 times). Wash the combined organic layers with 1M aqu HCl, water and brine, extract the combined aqu layers once again with EtOAc, dry the combined organic layers with Na₂SO₄ and remove solvent under reduced pressure. The left crude product is washed with ice cold EtOAc and dried in oil pump vacuum to obtain (40) as a white solid (2.28g, 83%). No further purification. [M. Nichifor; E. H. Schacht; *Tetrahedron;* **1994**; *50;* 12; 3747-3760]. ¹H NMR (400MHz, CDCl₃): 0.87-1.02 (br.m, 2 H); 1.34-1.52 (br.m, 4 H); 1.81 (br.d, 2 H, *J* ≈ 6Hz); 1.90-2.11 (br.s, 1 H); 2.12-2.35 (br.m, 1 H); 3.03 (Ψt, 2 H, *J* = 6.2Hz); 4.19 (br.Ψt, 1 H, *J* = 6.5Hz); 4.42 (br.Ψd, 2 H, *J* = 6.5Hz); 4.74 (br.s, 1 H); 7.30 (Ψt, 2 H, *J* = 7.4Hz); 7.38 (Ψt, 2 H, *J* = 7.4Hz); 7.57 (d, 2 H, *J* = 7.4Hz); 7.75 (d, 2 H, *J* = 7.4Hz).

**Step 2:** Dissolve (40) (190mg, 0.5mmol) in DCM (0.5mL) and DMF (0.25mL) at rt in a pre-dried tube, add DIEA (0.52mL, 1.5mmol) and add this solution to 2-chlorotritylchlorid-polystyrene (82mg, 0.13mmol) which is preswollen before in DCM (0.15mL). Shake the reaction suspensions for 14h at rt. The resin is (41) washed with DCM/MeOH/DIEA (17+2+1, 3 times), DCM (once), DMF (3 times) and again DCM (twice) and dried in vacuum. [i.e. *Novabiochem*® *2000 Catalog;* **2000;** S15-S18].

**Step 3:** Suspende complete amount of resin (41) from step 2 in DMF (0.4mL) and piperidine (0.1mL) and shake it for 1.5h at rt. Washed resin (42) with DMF (3 times) and DCM (3 times for 30min) and dry in vacuum.

**Step 4**: Complete amount of resin (42) from step 3 was preswollen in DMF (0.8mL) for 20min. Dissolve acid (43) (175mg, 0.59mmol) and HOBt (90mg, 0.59mmol) in DMF (2.8mL) at rt, add DIC (75mg, 0.59mmol), stir for 15min and add this solution to the preswollen resin (42). Shake the resin suspension gently for 20h at rt. Wash resin (44) with DMF (3 times) and DCM (4 times) and dry in vacuum.

**Step 5**: Suspend complete amount of resin (44) in 1.5mL of DCM+HFIP (2+1) and shake for 45min at rt. Filter the remaining solution of and wash resin once with DCM . Repeat cleavage procedure and washing once. Evaporate solvent of the combined filtrates under reduced pressure to affort crude product (45). No further purification.

**Step 6:** (The following reaction is done in an anhydrous N₂ atmosphere.) Suspende complete amount of crude (45) from step 3 in anhydrous DCM (2.0mL), cooled it to -40°C and added BBr₃ (0.15mL, 1.59mmol). Shake the reaction suspension for 1h at -40°C, 2h at - 25°C and 30min at +5°C. Add dropwise water under vigorous stirring followed by MeOH. Remove solvent under reduced pressure. Purify the crude product by preparative RP HPLC (gradient, water/MeCN 95:5) to obtain (46) (9mg, 17% over 5 steps) as a beige solid. ¹H NMR (400MHz, CD₃OD): 1.02 (dΨq, 2 H, *J*_{*1*} = 3.0Hz, *J*_{*2*} = 12.3Hz); 1.30-1.55 (m, 3 H); 1.86 (br.d, 2 H, *J* ≈ 13Hz); 1.94 (Ψquint, 2 H, *J* = 7.1Hz); 2.03 (br.d, 2 H, *J* ≈ 13Hz); 2.24 (ΨtΨt, 1 H, *J*_{*1*} = 3.3Hz, *J*_{*2*} = 12.3Hz); 2.31 (t, 2 H, *J* = 7.1Hz); 3.05 (d, 2 H, *J* = 6.6Hz); 3.43 (t, 2 H, *J* = 7.1Hz); 6.40 (d, 1 H, *J* = 8.8Hz); 7.16 (d, 1 H, *J* = 8.8Hz).

### Sialyl Lewis^{x} Tyrosine Sulfate Assay (sLe^{x} TSA):

Compounds of the present invention are assayed on a molecular level for their ability to inhibit the binding of P-, L-, or E-selectin chimeric molecules to sLe and tyrosinesulfate residues linked to a polymeric matrix as a PSGL-1 substitute. Selected 50% inhibitory concentrations (IC₅₀-values) are determined.
Microtiter plates are coated overnight in carbonate buffer pH9,6 with goat anti human Fc mAB (10 *µ*g/ml). After washing in assay buffer (25mM 4-(2-hydroxyethyl)-1-piperazineethanesulfonic acid (HEPES), 150mM NaCl, 1mM CaCl₂ pH7,4) and blocking (3% bovine serum albumin (BSA) in assay buffer) plates are incubated for 2h at 37°C with human P-Selectin-IgG-chimera (0,61nM respectively 150ng/mL) or human L-Selectin-IgG-chimera (0,61nM respectively 89ng/mL) or human E-Selectin-IgG-chimera (0,61nM respectively 131ng/mL). 5*µ*l of sLe^{x} -tyrosine sulfate polyacrylamide (lmg/ml) carrying 15% sLe^{x}, 10% Tyrosine-sulfate and 5% biotin is complexed with 20*µ*l Streptavidin-Peroxidase solution (1mg/ml) and 25*µ*l assay buffer without CaCl₂. For use in the assay, the ligand complex is diluted 1:10000 in assay buffer and further diluted 1:1 with varying amounts of compounds in assay buffer incl. 2%DMSO. This mixture is added to the wells precoated with P- or P-selectin. After incubation for 2h at 37°C, wells are washed for six times with in assay buffer incl. 0,005% Polyoxyethylenesorbitan monolaurate (TWEEN 20), developed for 10-15min with 20*µ*l 3,3',5,5'-tetramethylbenzidine (TMB)/H₂0₂ substrate solution and stopped with 20*µ*l 1M H₂SO₄. Bound sLe^{x} -Tyrosine sulfate ligand complex is determined by measuring optical density at 450nm vs. 620nm in a Fusion alpha-FP reader (sold from Packard Bioscience, Dreieich, Germany).

The compounds referred to in the following SCHEME11 are those compounds referred to as the particularly preferred compounds herein.

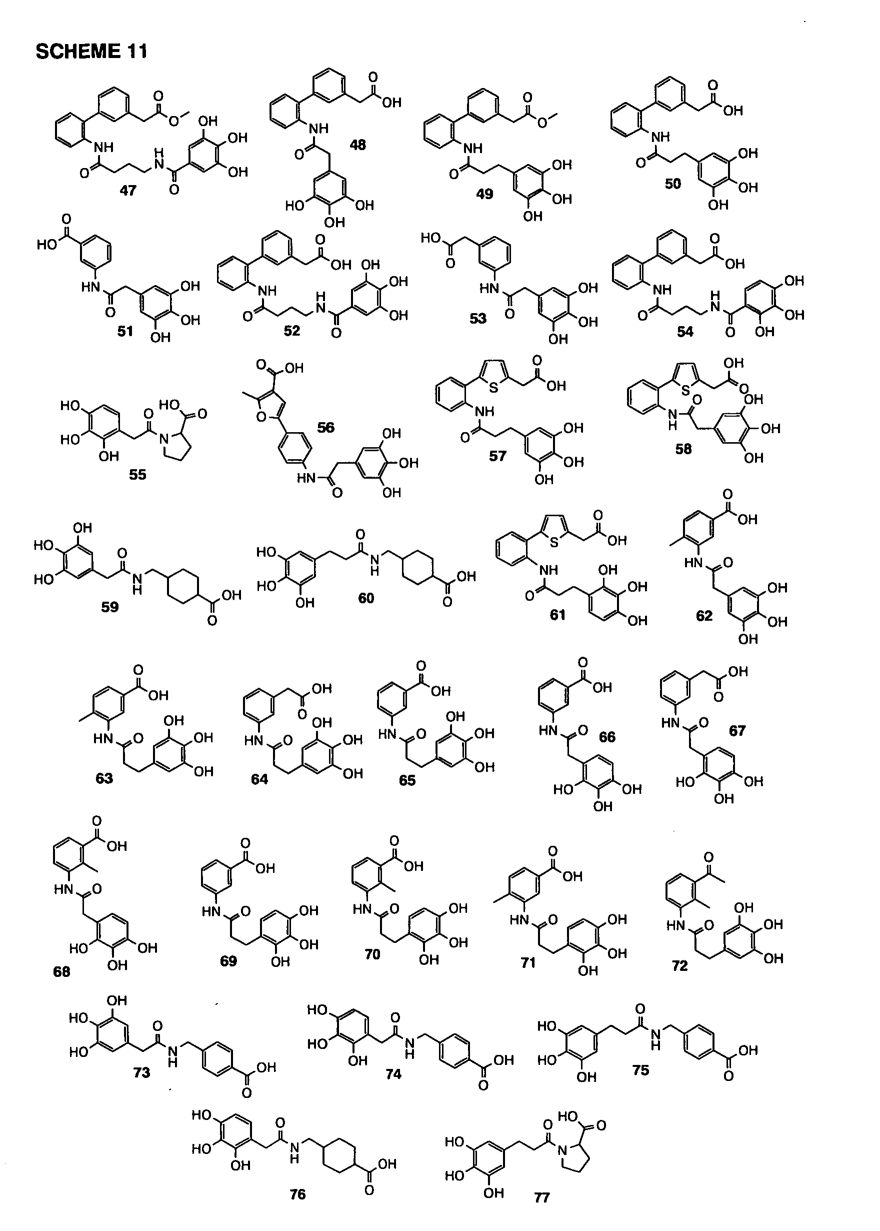

| **Table of sLe TSA Data; in** ***vitro*****.Inhibition of E-/P-/L-Selectin at 100** ***µ*****M** | | | | | | |
|---|---|---|---|---|---|---|
| **Compound** | **E-Selectin** **[% inhib.]** | **STD** | **P-Selectin** **[% Inhib.]** | **STD** | **L-Selectin** **[% inhib.]** | **STD** |
| 34 | 46.5 | 4.4 | 92.4 | 0.2 | 81.9 | 0.6 |
| 47 | 56.2 | 9.4 | 97.4 | 0.4 | 87.7 | 4.2 |
| 48 | 79.4 | 1.7 | 78.2 | 2.7 | 76.2 | 1.6 |
| 49 | 54.4 | 10.0 | 84.9 | 1.4 | 88.4 | 2.5 |
| 50 | 89.2 | 0.4 | 90.5 | 1.0 | 81.7 | 0.5 |
| 51 | 63.1 | 9.4 | 86.2 | 2.2 | 73.7 | 2.8 |
| 52 | 32.9 | 7.0 | 87.4 | 0.2 | 81.0 | 1.6 |
| 53 | 58.5 | 1.4 | 57.7 | 4.3 | 52.3 | 3.8 |
| 54 | 58.9 | 4.3 | 96.5 | 1.0 | 83.6 | 1.0 |
| 55 | 37.6 | 4.1 | 51.2 | 1.3 | 48.7 | 7.0 |
| 56 | 50.4 | 1.8 | 93.1 | 0.9 | 95.2 | 0.5 |
| 57 | 99.8 | 2.1 | 97.9 | 0.4 | 96.5 | 0.2 |
| 58 | 68.9 | 5.0 | 87.3 | 0.7 | 81.7 | 0.9 |
| 59 | 67.5 | 2.3 | 67.1 | 8.4 | 62.4 | 3.4 |
| 60 | 90.2 | 4.0 | 86.3 | 2.2 | 84.9 | 1.9 |
| 61 | 52 | 0.6 | 94.6 | 2.5 | 85.4 | 4.7 |
| 62 | 53.4 | 7.6 | 87.2 | 0.7 | 75.4 | 1.0 |
| 63 | 85.4 | 5.3 | 86.6 | 1.6 | 84 | 2.2 |
| 64 | 82.9 | 1.4 | 73.1 | 1.4 | 72.6 | 2.8 |
| 65 | 96.4 | 3.3 | 96.1 | 0.6 | 92 | 1.0 |
| 66 | 46 | 6.9 | 99.1 | 0.1 | 97.4 | 0.3 |
| 67 | 38.7 | 4.0 | 97.7 | 0.2 | 83.8 | 1.2 |
| 68 | 42.2 | 5.6 | 73.8 | 3.1 | 48.8 | 3.1 |
| 69 | 38.7 | 9.1 | 99.2 | 0.1 | 95.0 | 0.4 |
| 70 | n.a. | | 89.4 | 2.1 | 88.7 | 3.6 |
| 71 | 92.7 | 2.8 | 88.6 | 1.9 | 87.4 | 1.5 |
| 72 | 94.9 | 5.1 | 86.6 | 3.7 | 89.9 | 3.9 |
| 73 | 53.5 | 0.7 | 79.4 | 2.8 | 66.2 | 3.2 |

| **Results from sLe**^{**x**}**TSA: IC**_{**50**} **Data for E-/ P-/ L-Selectin** | | | | | | |
|---|---|---|---|---|---|---|
| **Compound** | **IC**_{**50**} **E-Selectin [µM]** | **STD** | **IC**_{**50**} **P-Selectin [µM]** | **STD** | **IC**_{**50**} **L-Selectin [µM]** | **STD** |
| 34 | n.a. | | 7.7 | 1.0 | 11.6 | 3.3 |
| 51 | 0.8 | 0.2 | 1.1 | 0.0 | 1.4 | 0.3 |
| 53 | 1.0 | 0.7 | 2.1 | 0.6 | n.a. | |
| 57 | 3.3 | 2.0 | 2.4 | 0.7 | 3.1 | 1.0 |
| 59 | 3.0 | 1.2 | 2.8 | 1.1 | 3.3 | 0.9 |
| 60 | 3.1 | 2.4 | 3.4 | 2.1 | 5.7 | 1.0 |
| 63 | 1.3 | 0.3 | 1.7 | 0.5 | 1.8 | 0.2 |
| 64 | 2.3 | 1.0 | 2.3 | 0.6 | 2.3 | 0.7 |
| 66 | 0.6 | 0.2 | 2.1 | 0.7 | 7.0 | 7.7 |
| 70 | n.a. | | 7.4 | 1.0 | 16.0 | 4.8 |
| 71 | 7.3 | 9.4 | 13.0 | 17.2 | 15.0 | 20.0 |
| 72 | 8.4 | 5.6 | 21.7 | 25.7 | 109.4 | 198.8 |
| 73 | 12.2 | 16.0 | 14.8 | 13.9 | 26.2 | 32.5 |

### Flow Chamber Assay / Cell Adhesion and Rolling under Flow Conditions

To assess the capability of compounds to inhibit cell binding under dynamic conditions resembling the flow in a blood vessel, flow chamber assays addressing/ testing binding of HL-60 cells / various cell lines to P-selectin, L-selectin and E-selectin chimeric molecules are performed.
Cell attachment under flow conditions are determined using a parallel flow chamber system. A 35mm polystyrene culture dish is coated for 1 hour at room temperature with coating buffer (50mM tris-(hydroxymethyl) aminomethane buffer (Tris), 150 mM NaCl, 2 mM CaCl₂; pH 7,4) containing human E- or P-selectin-IgG chimera at concentrations of 2,5µg/ml or 10µg/ml, respectively. After removal of the coating solution non specific binding sites are blocked for an additional hour with 1% BSA in coating buffer at room temperature. After washing with assay buffer ("Roswell Park Memorial Institute 1640" (RPMI 1640) + 10mM HEPES) the dish is fitted into a parallel plate laminar flow chamber (sold from Glycotech, Rockville, MD) and mounted on an inverted phase-contrast microscope (sold from Olympus, Hamburg, Germany) equipped with a CCD camera (JVC) that is connected to a PC. Employing a peristaltic pump (sold from Ismatec, Wertheim-Mondfeld, Germany) the re-circulating system is equilibrated with assay buffer containing 125µM compound or vehicle control (DMSO). Cells (1 million / ml) are added to the chamber and allowed to distribute for 2 minutes at a high flow rate. The flow rate is then decreased resulting in a calculated flow shear of 1 dyne/cm². Video sequences of 10 low power fields are digitally recorded after 5 minutes continuous flow. The percentage of inhibition is calculated from the mean number of cells per field that attached to the coated dish surface in the presence versus absence of compound of at independent experiments.

| **Data from Flow Chamber Assay for E- and P-Selectin** | | |
|---|---|---|
| **Compound** | **E-Selectin [% inhib.]** | **P-Selectin [% inhib.]** |
| 57 | 35 | 11 |
| 63 | n.a. | 17 |
| 71 | 22 | 22 |

## Claims

1. Compounds having the general structure of formula (I), 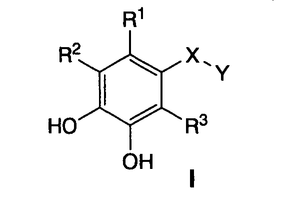 wherein the symbols, indices and substituents have the following meaning
if R²=OH and R³=H then R¹=H, CN, NO₂, CF₃, F, Cl, Br, I, CH₃ or
if R³=OH and R²=H then R¹=H, CN, NO₂, CF₃, F, Cl, Br, I, CH₃, Et, n-Pr, i-Pr, n-Bu, i-Bu, t-Bu, phenyl, thienyl, furyl, thiazolyl or
if R³=OH and R¹=H then R²=H, CN, NO₂, CF₃, F, Cl, Br, I, CH₃, Et, n-Pr, i-Pr,
n-Bu, t-Bu, phenyl, thienyl, furyl, thiazolyl
-X-=
(a) 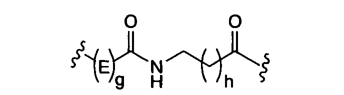 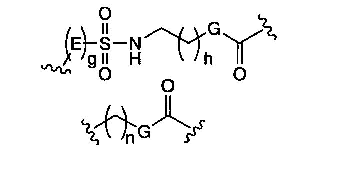 with -E- being -NH-, -(CH₂-)ₖNH-; -G- being -(NH-)ₘ and g = 0,1; h = 1, 2, 3; k= 1, 2, 3; m = 0,1; n = an integer from 1 to 8
(b) 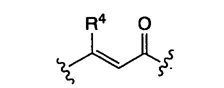 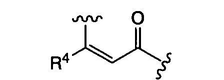 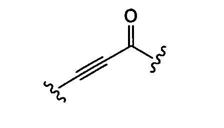 with R⁴ being H, CH₃, CH₂CH₃
(c) 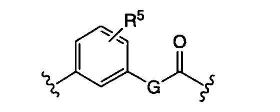 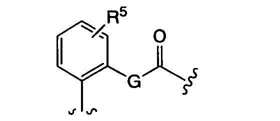 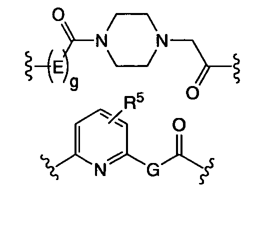 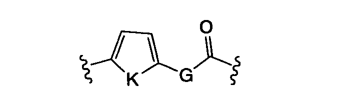 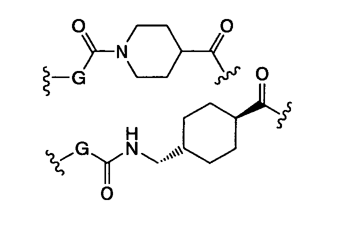 with R⁵ being H, NO₂, CF₃, F, Cl, Br, I, CN, CH₃ and -K- being -S- or -O-
(d) 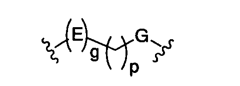 with p being an integer from 2 to 8
(e) 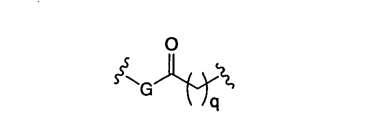 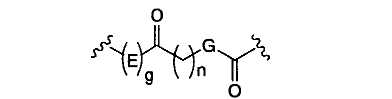 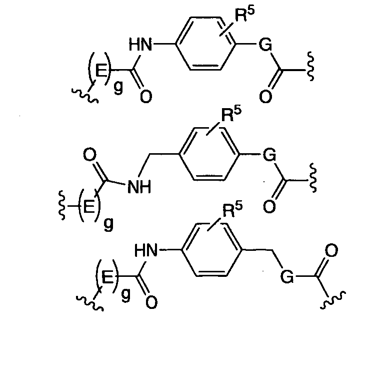 with q being an integer from 1 to 9 and r being an integer from 1 to 3
(f) 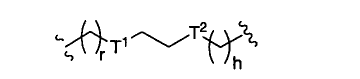 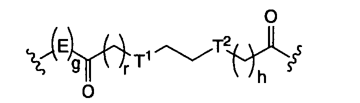 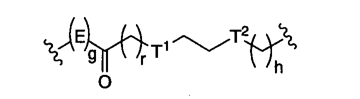 with -T¹-, -T²-being -E-, -K- or -(N-alkyl)-
-Y = 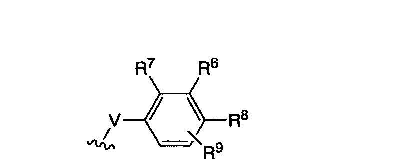 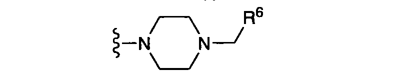 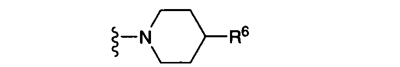 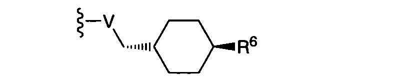 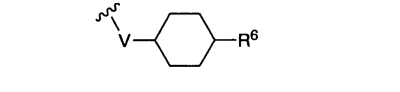 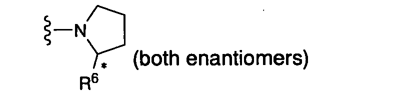 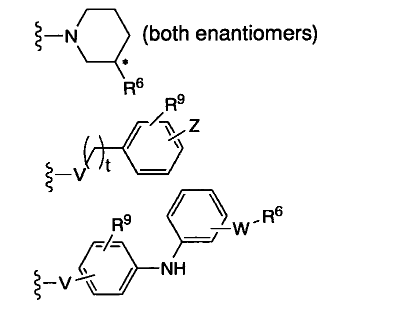 with -V- being -(NH-)ₛ- with s being 0 or 1,
R⁶ being CO₂H, CO₂Alkyl, CO₂Aryl, CO₂NH₂, CO₂Aralkyl, SO₃H, SO₂NH₂, PO(OH)₂, 1-H-tetrazolyl-, CHO, COCH₃, CH₂OH, NH₂, NHAlkyl, N(Alkyl)Alkyl', OCH₃, CH₂OCH₃, SH
R⁷ independently from R⁶ being H, CH₃, CH₂CH₃, CF₃, F, Cl, Br, I, CN, NO₂ and
R⁸ independently from R⁶ and R⁷ being H, CH₃, CH₂CH₃, CF₃, F, Cl, Br, I, CN, NO₂, R⁶
R⁹ being H, NO₂, CF₃, F, Cl. Br, I, CN, CH₃, OCH₃, SH
t being 0,1,2
and -W- = -(CH₂-)ᵥ, *cis*-CH=CH- or *trans*-CH=CH-, and v being 0,1,2
-Z = 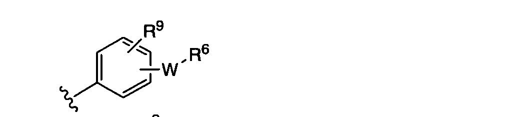 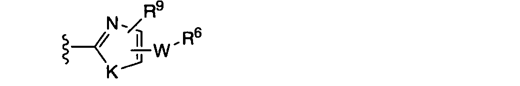 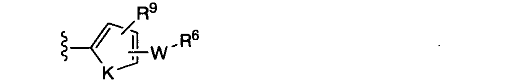 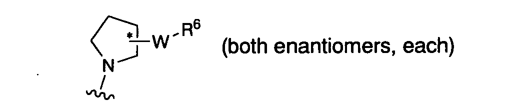
-W-R⁶
and the pharmaceutically acceptable salts, esters or amides and prodrugs of the above identified compounds of formula (I).

2. Compounds according to claim 1, wherein the compounds are defined by formulas (A) and (B) 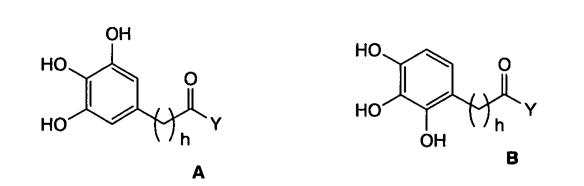 wherein -Y is (Q=CH, N) 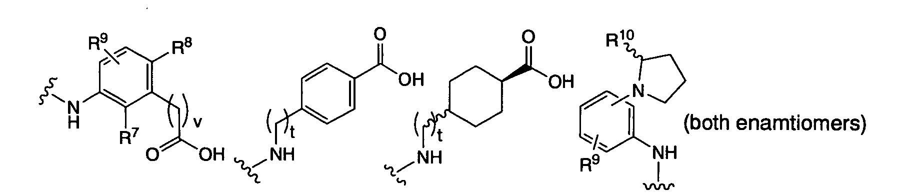 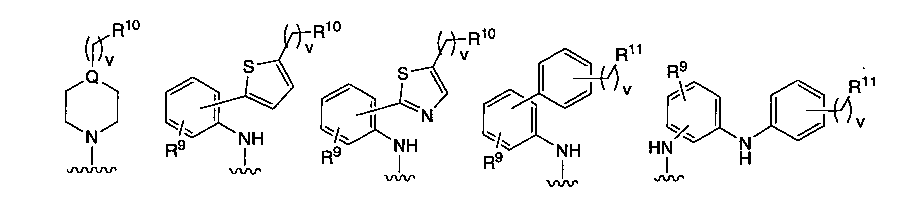 with R¹⁰ being CO₂H, CO₂alkyl, CO₂aryl, CO₂NH₂, CO₂aralkyl, CH₂SO₃H, CH₂SO₂NH₂, CH₂PO(OH)₂, 1-H-tetrazolyl, CHO, COCH₃, CH₂OH, CH₂NH₂, CH₂NHalkyl, CH₂N(alkyl)alkyl', CH₂OCH₃, CH₂SH,
R¹¹ being CO₂H, CO₂alkyl, CO₂aryl, CO₂NH₂, CO₂aralkyl, SO₃H, SO₂NH₂, PO(OH)₂, 1-H-tetrazolyl, CHO, COCH₃, OH, NH₂, NHalkyl, N(alkyl)alkyl', OCH₃, SH.

3. Compounds according to claim 1, wherein the compounds are defined by formulas (C) and (D) 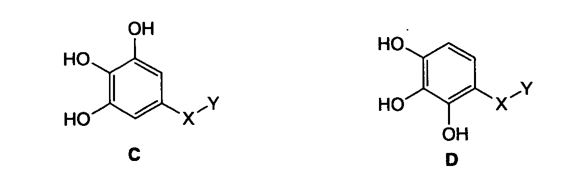 wherein Y is like relating to formulas (A) and (B) and X is:

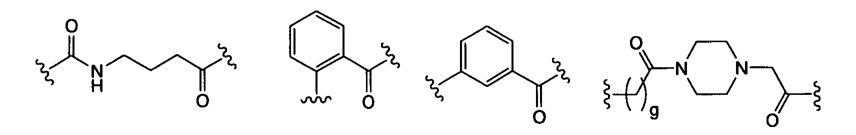

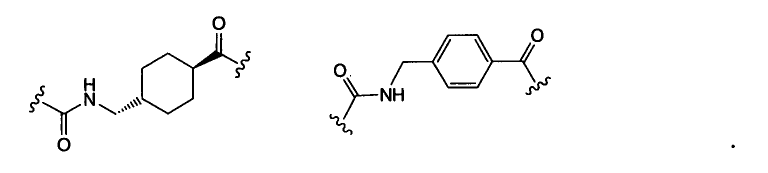

4. Pharmaceutical compositions comprising at least one compound of the formala (I) and a pharmaceutically acceptable carrier which is useful in a medicine.

5. Pharmaceutical compositions according to claim 4, comprising at least one compound of formula (A) or formula (B) or formula (C) or formula (D).

6. Method of inhibiting the binding of P-selectin, L-selectin or E-selectin to sLe^{x} or sLe^{a} and tyrosinesulfate residues comprising the step of administering to a patient an effective amount of at least one compound having the structure of formula (I).

7. Use of compounds according to any one of claims 1 to 3 for the preparation of a medicine for the treatment of a patient, inhibiting the binding of P-selectin, L-selectin or E-selectin to sLe^{x} or sle^{a} and tyrosinesulfate residues.
